# EUROPEAN PATENT SPECIFICATION

(11) **EP 2 675 784 B1**
(45) Date of publication and mention of the grant of the patent: **13.09.2017**
(21) Application number: 12707196.7
(22) Date of filing: 17.02.2012
(51) Int. Cl.: C07C 303/08, C07C 309/31, C07C 6/04, C07C 1/24, C07C 11/04

(54) **BIO-BASED LINEAR ALKYLPHENYL SULFONATES**
BIOBASIERTE LINEARE ALKYLPHENYLSULFONATE
SULFONATES D'ALKYLPHÉNYLE LINÉAIRES D'ORIGINE BIOLOGIQUE

(30) Priority: 17.02.2011 US 201161443981 P
(43) Date of publication of application: 25.12.2013
(73) Proprietor: The Procter & Gamble Company, Cincinnati, OH 45202 (US)
(72) Inventor: GREEN, Phillip, Richard, Wyoming, Ohio 45231 (US); SCHEIBEL, Jeffrey, John, Loveland, Ohio 45140 (US); COLLIAS, Dimitris,Ioannis, Mason, Ohio 45040 (US)
(74) Representative: Peet, Jillian Wendy
(86) International application number: PCT/US2012/025540
(87) International publication number: WO 2012/112828

(56) References cited:
- WO-A1-99/05084
- WO-A1-2009/140695
- WO-A1-2010/062958
- US-A1- 2010 145 086
- US-A1- 2010 191 008
- J.J. SCHEIBEL: "The evolution of anionic surfactant technology to meet the requirements of the laundry detergent industry", JOURNAL OF SURFACTANTS AND DETERGENTS, vol. 7, no. 4, October 2004 (2004-10), pages 319-328, XP055033609, Springer, Berlin, DE ISSN: 1097-3958, DOI: 10.1007/s11743-004-0317-7 cited in the application

## Description

### FIELD OF THE INVENTION

The invention is directed to a method of making a a mixture of C₁₀-C₁₄ linear alkylphenyl sulfonates having alkyl groups that each have a biobased content of at least 50%.

### BACKGROUND OF THE INVENTION

Surfactants are the single most important cleaning ingredient in cleaning products. Environmental regulations, consumer habits, and consumer practices have forced new developments in the surfactant industry to produce lower-cost, higher-performing, and environmentally friendly products. Examples of developments in the surfactant industry are described by J. Scheibel in the Journal of Surfactants and Detergents, "The Evolution of Anionic Surfactant Technology to Meet the Requirements of the Laundry Detergent Industry," volume 7, number 4, October, 2004, pages 319-328 ("Scheibel JSD Article" hereinafter). Today, challenges facing the surfactant industry include colder wash temperatures, less efficient builders, liquid products without calcium control, and a push for reduced surfactant use overall because of the perceived environmental impact of surfactants.

Alkylphenyl sulfonates are surfactants derived from tetrapropylene that have very complex branching structures (e.g., 3 or 4 branches per molecule). ABS surfactants were prominent until the early 1960s when they were subjected to environmental regulations for being poorly biodegradable. Alkylphenyl sulfonate surfactants were then replaced with the readily biodegradable linear alkylphenyl sulfonate (LAS) surfactants, which are easily obtainable and currently in use today. Use of LAS surfactants and other similar linear surfactants is limited because they have poor solubility in cold water and hard water conditions. In fact, more than half of the LAS detergent in products may be lost during use due to the formation of multilayered vesicles that resemble large onion-like structures. Formulators can increase the solubility of linear surfactants, for example, by introducing cosurfactants or by using linear alcohol ethoxylated sulfates (AES). However, AES surfactants have lower surface activity, as well as lower mass efficiency than LAS surfactants. Further, the use of cosurfactants or AES surfactants limits formulation flexibility and can add substantial cost to the detergent. ABS, LAS, and AES surfactants are described in detail in the Scheibel JSD article.

The materials used to produce LAS surfactants are derived from non-renewable resources, such as petroleum, natural gas, and coal. As used herein, "renewable resource" refers to one that is produced by a natural process at a rate comparable to its rate of consumption (e.g., within a 100 year time frame). The resource can be replenished naturally, or via agricultural techniques. Nonlimiting examples of renewable resources include plants (e.g., sugar cane, beets, corn, potatoes, citrus fruit, woody plants, lignocellulosics, hemicellulosics, cellulosic waste), animals, animal fats, fish, bacteria, fungi, plant-based oils, and forestry products. These resources can be naturally occurring, hybrids, or genetically engineered organisms. Natural resources such as crude oil, natural gas, coal, and peat, which take longer than 100 years to form, are examples of non-renewable resources. As used herein, "petroleum" refers to crude oil and its components of paraffinic, cycloparaffinic, and aromatic hydrocarbons. Crude oil may be obtained from tar sands, bitumen fields, and oil shale.

Thus, the price and availability of the petroleum, natural gas, and coal feedstock ultimately have a significant impact on the price of LAS surfactants. As the worldwide price of petroleum, natural gas, and/or coal escalates, so does the price of LAS surfactants and compositions made using LAS surfactants. Furthermore, these resources have finite limitations for the future as they are not replenished. Some reports indicate crude oil reserves are less than 100 years, or even less than 30 years. Many consumers display an aversion to purchasing products that are derived from petrochemicals. In some instances, consumers are hesitant to purchase products made from limited non-renewable resources (e.g., petroleum, natural gas, and coal). Other consumers may have adverse perceptions about products derived from petrochemicals as being "unnatural" or not environmentally friendly. Some consumers are concerned for future generations, that they will not have the lifestyle that petroleum resources have provided the current generation. Thus, alternatives are needed that are renewably sourced but at reasonable economics and processed in a way that uses less energy to prepare.

Accordingly, it would be desirable to provide LAS surfactants using monomers derived from renewable resources.

### SUMMARY OF THE INVENTION

The invention is directed to a method of making a mixture of C₁₀-C₁₄ linear alkylphenyl sulfonates having a controlled total carbon atom distribution comprising the steps of:
(a) dehydrating a C₁₀-C₁₄ alcohol mixture derived either from plant matter or a bioengineered microorganism to form a mixture of C₁₀-C₁₄ alkenes; wherein each alkene has a biobased content of at least 50%;
(b) optionally isolating the C₁₀-C₁₄ alkenes;
(c) alkylating benzene with the mixture of C₁₀-C₁₄ alkenes to form a mixture comprising C₁₀-C₁₄ linear alkylbenzenes; and
(d) sulfonating the mixture of C₁₀-C₁₄ linear alkylbenzenes to form a mixture comprising C₁₀-C₁₄ linear alkylphenyl sulfonates.

The invention is directed to a method of making a mixture comprising (a) C₁₀-C₁₄ linear alkylphenyl sulfonates. The alkyl groups of the alkylphenyl sulfonates each independently have a total of 10-14 carbon atoms, and each alkyl group has a biobased content of at least about 5%, at least about 10%, at least about 20%, at least about 30%, at least about 40%, at least about 50%, at least about 60%, at least about 70%, at least about 80%, at least about 90%, at least about 95%, for example, about 100%. Further, the mixture comprises less than about 5 wt.% of (1) linear alkylbenzenes or linear alkylphenyl sulfonates that have alkyl groups with 9 or fewer carbon atoms and alkyl groups that have 15 or more carbon atoms, based on the total weight of the mixture; (2) C₁₀-C₁₄ linear alkylbenzenes or C₁₀-C₁₄ linear alkylphenyl sulfonates having two or more C₁₀-C₁₄ alkyl groups on the benzene or on the phenyl group; and (3) C₁₀-C₁₄ linear alkylbenzenes or C₁₀-C₁₄ linear alkylphenyl sulfonates having an alkyl group comprising a quaternary carbon atom. The benzene portion of the C₁₀-C₁₄ linear alkylbenzenes or the phenyl moiety in the C₁₀-C₁₄ linear alkylphenyl sulfonates in the mixtures described each optionally have a biobased content of at least about 50%, preferably at least about 70% or at least 90%, more preferably at least about 95%, for example about 100%. In some embodiments, C₁₀ alkyl is present in 22 wt%, C₁₁ alkyl is present in 31.9 wt%, C₁₂ alkyl is present in 29.6 wt%, C₁₃ alkyl is present in 16.1 wt%, and C₁₄ alkyl is present in 0.3 wt%. In various embodiments, C₁₀ alkyl is present in 10 wt%, C₁₁ alkyl is present in 37.2 wt%, C₁₂ alkyl is present in 32.3 wt%, C₁₃ alkyl is present in 19.9 wt%, and C₁₄ alkyl is present in 0.7 wt%. In still other embodiments, C₁₀ alkyl is present in 13 wt%, C₁₁ alkyl is present in 30.5 wt%, C₁₂ alkyl is present in 31.5 wt%, C₁₃ alkyl is present in 24.3 wt%, and C₁₄ alkyl is present in 0.7 wt%. In some cases, C₁₀ alkyl is present in 9.3 wt%, C₁₁ alkyl is present in 21.0 wt%, C₁₂ alkyl is present in 25.6 wt%, C₁₃ alkyl is present in 30.7 wt%, and C₁₄ alkyl is present in 13.5 wt%.

A consumer product cleaning or personal care composition may comprise about 0.001 wt.% to about 99.999 wt.%, preferably about 0.1 wt% to about 80 wt.%, of the mixture of C₁₀₋C₁₄ linear alkylphenyl sulfonates, as described herein, based on the total weight of the composition, and 0.001 wt.% to about 99.999 wt.% of one or more additional cleaning components, or one or more additional personal care components. In various embodiments, the at least one cleaning component is selected from the group consisting of a surfactant, an enzyme, a builder, an alkalinity system, an organic polymeric compound, a hueing dye, a bleaching compound, an alkanolamine, a soil suspension agent, an anti-redeposition agent, a corrosion inhibitor, and a mixture thereof. In some cases, the composition is selected from the group consisting of a granular detergent, a bar-form detergent, a liquid laundry detergent, a liquid hand dishwashing composition, a hard surface cleaner, a tablet, a disinfectant, an industrial cleaner, a highly compact liquid, a powder, and a decontaminant. In a class of cases, the composition is enclosed within a sachet or a multi-compartment pouch comprising both solid and liquid compartments.

The at least one personal care component is selected from the group consisting of an oil, and emollient, a moisturizer, a carrier, an extract, a vitamin, a mineral, an anti-aging compound, a surfactant, a solvent, a polymer, a preservative, an antimicrobial, a wax, a particle, a colorant, a dye, a fragrance, and mixtures thereof. In various cases, the composition is a shampoo, a hair conditioner, a hair treatment, a facial soap, a body wash, a body soap, a foam bath, a make-up remover, a skin care product, an acne control product, a deodorant, an antiperspirant, a shaving aid, a cosmetic, a depilatory, a fragrance, and a mixture thereof. In a class of cases, the composition is delivered in a form selected from the group consisting of a wipe, a cloth, a bar, a liquid, a powder, a crème, a lotion, a spray, an aerosol, a foam, a mousse, a serum, a capsule, a gel, an emulsion, a doe foot, a roll-on applicator, a stick, a sponge, an ointment, a paste, an emulsion spray, a tonic, a cosmetic, and mixtures thereof. In various embodiments, the composition further comprises a product selected from the group consisting of a device, an appliance, an applicator, an implement, a comb, a brush, a substrate, and mixtures thereof. In some embodiments, the composition is dispensed from an article selected from the group consisting of a bottle, a jar, a tube, a sachet, a pouch, a container, a tottle, a vial, an ampoule, a compact, a wipe, and mixtures thereof.

The alcohol mixture for dehydration may be made by hydroformylation and subsequent reduction of alkenes that result from the metathesis reaction. In the method of making a mixture of renewable C₁₀-C₁₄ alkenes, a fatty acid, a fatty ester, a fat, an oil, or mixtures thereof is reacted with an alkene having a total of 2 to 8 carbon atoms in the presence of a catalytically effective amount of a metathesis catalyst and under standard metathesis conditions. The fatty acid, fatty ester, fat, oil, or mixture thereof has an iodine value of at least about 15, as determined by the AOAC Official Method of Analysis (1984), Chapter 28.023. The mixture of alkenes produced by the metathesis method described herein has a biobased content of at least about 5 wt.% %, at least about 10%, at least about 20%, at least about 30%, at least about 40%, at least about 50%, at least about 60%, at least about 70%, at least about 80%, at least about 90%, at least about 95%, for example, about 100%.

All percentages, ratios and proportions herein are by weight, unless otherwise specified. All temperatures are in degrees Celsius (°C) unless otherwise specified.

### DETAILED DESCRIPTION

The invention is directed to a method of making C₁₀-C₁₄ linear alkylphenyl sulfonates, wherein each alkyl group has a biobased content of at least 50%. The mixtures of C₁₀-C₁₄ linear alkylphenyl sulfonates are biodegradable, completely tunable (e.g., by altering the alkyl chain distribution), and can be used in consumer product cleaning and personal care cleaning compositions. These mixtures of C₁₀-C₁₄ linear alkylphenyl sulfonates, or the C₁₀-C₁₄ linear alkylbenzene intermediates used to make the C₁₀-C₁₄ linear alkylphenyl sulfonates, also can be spiked into existing, petroleum-derived alkylbenzenes or alkylphenyl sulfonates.

In the method of the invention, a C₁₀-C₁₄ alcohol mixture derived either from plant matter or a bioengineered microorganism is dehydrated to form a mixture of C₁₀-C₁₄ alkenes; wherein each alkene has a biobased content of at least 50%; and optionally isolating the of C₁₀-C₁₄ alkenes. The resulting mixture of C₁₀-C₁₄ linear alkenes is used to alkylate benzene to form a mixture of renewable C₁₀-C₁₄ alkylbenzenes. These alkylbenzenes then are sulfonated to form the mixture of C₁₀-C₁₄ alkylphenyl sulfonates.

The metathesis process can be used to replace traditional methods for the production of linear alkylphenyl sulfonates that use kerosene and/or other petroleum based feedstock. The traditional kerosene process is inefficient and requires multiple process steps to convert kerosene to linear paraffins. Further, only about 20% of the alkenes that are produced by the process undergo alkylation, requiring recycling of the paraffin back into the dehydrogenation process, a very energy intensive process. Because plants that produce linear alkylbenzenes are large with large recycle streams, about 1000 KMTA of kerosene is needed to run the plant, while only about 150 KMTA of linear alkylbenzenes are produced. In contrast, the metathesis process of the invention has increased throughput in alkylation per unit of starting material, making the cost savings that is available for new plants economically competitive with the traditional system. Further, simple stirred batch reactors can be used for the method of the invention without any special steel requirement. As a result, an affordable multiple reactor system can be used that allows the composition of the products of metathesis to be tailored by running multiple batch reactors with different short chain alkene feeds, and then post-blending the resulting alkenes to economically make up the mixtures of the invention. The ability to use multiple batch reactors with different short chain alkene feeds currently is not feasible with the previously described petroleum-based process because the kerosene is a gross mixture of components that have different chain lengths and different branching. Controlling or selecting either the total number of carbon atoms in the chain or type of branching of the chain is challenging and costly.

The metathesis process is advantageous because the feedstock can include crude starting materials that contain, for example, paraffin, isoparaffin, and aromatic contaminants because these contaminants are unreactive during the metathesis process, yet can easily be distilled from the product. Further still, the metathesis route has a simple reaction design with a minimum number of reaction steps, avoiding the use of complex fractionation of fuel feedstocks, which is cost prohibitive, as well as three of the complex and costly steps that are commercially used to synthesize petroleum-based linear alkylphenyl sulfonates (i.e., hydrogenation of kerosene, extraction of linear paraffins, and dehydrogenation of linear paraffins), as described above. It results in a clean mixture of C₁₀-C₁₄ alkenes that are easily separable from the triglyceride side products through simple distillation, without requiring extensive fractional distillation. This clean mixture of alkenes can, in some situations, be directly reacted in the alkylation reaction to form alkylbenzenes without substantial work-up or purification (e.g., sieving, concentration). In other situations, the metathesis derived alkenes can contain some di- or tri-alkenes, possibly requiring partial hydrogenation to mono-alkenes to limit the formation of undesirable tetralins and/or indans during the alkylation process. These impurities (e.g., tetralins and indans) are not as biodegradable as linear alkylbenzenes and linear alkylphenyl sulfonates.

Using metathesis chemistry to produce a mixture of C₁₀-C₁₄ alkenes with a controlled total carbon atom distribution allows for tunability of the C₁₀-C₁₄ alkene distribution through the selection of the short chain alkene starting material. For example, a formulator can select short chain alkene starting materials having a particular chain length distribution to meet the criteria of different cleaning composition formulations (e.g., dishwashing liquids, liquid laundry detergent, granular detergent). The formulator also can select alkyl chains having a particular degree and location of branching to meet biodegradability needs (e.g., some lightly branched alkylbenzenes and lightly branched alkylphenyl sulfonates have improved biodegradability).

The feedstock for the metathesis reaction of the invention (e.g., fatty acids, fatty esters, fats, oils, short chain alkenes) is also advantageous over petroleum feedstocks for the formation of mixtures of C₁₀-C₁₄ alkenes. Current feedstocks from petroleum resources have some short chain contamination (e.g, C₈, C₉) and some C₁₅ content depending on the average chain length of the paraffin cut from kerosene. This short chain contamination is carried through the subsequent alkylation step to form alkylbenzenes having short chain and long chain contaminants. If incomplete sulfonation occurs during the surfactant making process, these short chains can affect the volatile organic carbon (VOC) in spray tower detergent processing. Some states require substantial emission control to limit release of such VOC and can be subject to fines if these limits are exceeded in a processing plant performing sulfonation or spray tower drying of surfactants. In contrast, short chain contamination cannot exist in the process of the invention. Furthermore, the feedstock for this metathesis process (e.g., fatty acids, fatty ester, fats, oils, short chain alkenes) can be obtained at low cost.

The metathesis process generates three value added products: glycerin, alkene-terminated and near terminal olefinic acids and esters, and bio-alkene feedstock. Glycerin is commercially used, for example, for solvents and foods. Some saturated fatty esters can be present, depending upon the degree of unsaturation of the ester, acid, or oil used in the processing. These remain unreacted by metathesis and are also value add by-products but of lesser value than the olefins or short chain unsaturated esters, acids, or oils. Alkene-terminated and near terminal olefinic acids and esters can be commercially used for specialty applications, such as antimicrobials, polymer crosslinkers, and the generation of unique diacids, as described in the following presentations: Cargill, "Cargill's Activities to Develop Industrial Chemicals From Plants," Plant Bio-Industrial Oils Workshop, February 2006, and Elevance, "Novel Renewable Chemicals, Transforming Markets with New Buidling Blocks, March 2010. Bio-alkene feedstock can be used to form the linear alkylphenyl sulfonates of the invention or to form linear or branched bio-alcohols via hydroformylation and subsequent reduction.

The particular alkyl chain distributions of the mixtures of C₁₀-C₁₄ linear alkylbenzenes and alkylphenyl sulfonates are themselves advantageous during the formation of C₁₀-C₁₄ linear alkylphenyl sulfonates because they simplify the purification process. Typically, a difference in chain length of three carbon atoms (e.g., C₈ versus C₁₁) is necessary to allow purification by simple distillation. Separating chains without a difference in length of three carbon atoms (e.g., C₉ versus C₁₀) is not only extremely difficult, but also cost prohibitive. The alkyl distributions of the invention have a great enough difference in chain length to avoid difficult and costly separation techniques.

As used herein, "biobased content" refers to the amount of bio-carbon in a material as a percent of the weight (mass) of the total organic carbon in the product. For example, ethylene contains two carbon atoms. If ethylene is derived from a renewable resource, it has a biobased content of 100% because all of the carbon atoms are derived from a renewable resource. As another example, undecylbenzene contains 17 carbon atoms (i.e., 11 from the undecyl alkyl chain and 6 from the phenyl group). If the undecyl group is derived from a renewable resource, but the phenyl group is derived from a petroleum-based resource, the theoretical biobased content of the undecylbenzene is about 65%.

As used herein, a "renewable" compound or material is one that is partially or wholly derived from a renewable resource. In a partially renewable compound or material, at least one, but not all of its carbon atoms is derived from a renewable resource. In a wholly renewable compound or material, all of its carbon atoms are derived from a renewable resource.

As used herein, a "renewable resource" is one that is produced by a natural process at a rate comparable to its rate of consumption (e.g., within a 100 year time frame). The resource can be replenished naturally, or via agricultural techniques. Renewable resources include plants (e.g., sugar cane, beets, corn, potatoes, citrus fruit, woody plants, lignocellulosics, hemicellulosics, cellulosic waste), animals, fish, bacteria, fungi, and forestry products. These resources can be naturally occurring, hybrids, or genetically engineered organisms. Natural resources such as crude oil, coal, and peat, which take longer than 100 years to form, are not considered renewable resources. Nonlimiting examples of renewable polymers include polymers produced directly from organisms, such as polyhydroxyalkanoates (e.g., poly(beta-hydroxyalkanoate), poly(3-hydroxybutyrate-co-3-hydroxyvalerate, NODAX^{™}), and bacterial cellulose; polymers extracted from plants and biomass, such as polysaccharides and derivatives thereof (e.g., gums, cellulose, cellulose esters, chitin, chitosan, starch, chemically modified starch), proteins (e.g., zein, whey, gluten, collagen), lipids, lignins, and natural rubber; and polymers derived from naturally sourced monomers and derivatives, such as bio-polyethylene, polytrimethylene terephthalate, polylactic acid, NYLON 11, alkyd resins, and succinic acid-based polyesters.

The term "bio-" placed as a prefix means that at least a portion of the carbon atoms of the component are derived from a renewable resource. Also included within this definition are those components that are produced naturally in plants. For example, bio-limonene and bio-isobornyl alcohol can be harvested from various plants. While the component may be capable of being derived from petroleum feedstock, the prefix is intended to exclude those components that specifically derive all of their carbon atoms from petroleum feedstock. As an example, "bio-ethanol" means ethanol that is formed from renewable resources. Catalysts, solvents, or other adjuvants that are used to facilitate the reaction, but do not form a part of the final bio-component, do not necessarily need to be derived from a renewable resource.

As used herein, the term "biodegradable" refers to compounds and materials that are capable of undergoing natural decomposition into carbon dioxide, methane, water, inorganic compounds, biomass, or a mixture thereof, in which the predominant mechanism is the enzymatic action of microorganisms that can be measured by standardized tests, in a specified time, reflecting relevant disposal conditions. In the presence of oxygen (aerobic biodegradation), these metabolic processes yield carbon dioxide, water, biomass, and minerals. Under anaerobic conditions (anaerobic biodegradation), methane may additionally be produced.

As used herein, the term "alkyl" refers to straight chain saturated hydrocarbon groups, nonlimiting examples of which include methyl, ethyl, propyl, butyl, pentyl, hexyl, heptyl, and octyl groups containing the indicated number of carbon atoms. The term Cₙ means the alkyl group has "n" carbon atoms. For example, (C₁-C₇)alkyl refers to an alkyl groups having a number of carbon atoms encompassing the entire range (i.e., 1 to 7 carbon atoms), as well as all subgroups (e.g., 1-6, 2-7, 1-5, 3-6, 1, 2, 3, 4, 5, 6, and 7 carbon atoms).

As used herein, a "C₁₀-C₁₄ alkene" is a monounsaturated or unconjugated, polyunsaturated hydrocarbon having 10 to 14 total carbon atoms (e.g., 10, 11, 12, 13, or 14 total carbon atoms, as well as all subgroups, such as 10-14, 10-13, 10-12, 10-11, 11-14, 11-13, 11-12, 12-14, 12-13 total carbon atoms).

### Mixtures of Renewable C₁₀-C₁₄ Alkylphenyl Sulfonates

In another aspect, the alkyl groups on the linear alkylphenyl sulfonates each independently have a total of 10, 11, 12, 13, or 14 carbon atoms. The alkyl groups on the linear alkylphenyl sulfonates have a biobased content of at least about 5%, at least about 10%, at least about 20%, at least about 30%, at least about 40%, at least about 50%, at least about 60%, at least about 70%, at least about 80%, at least about 90%, at least about 95%, for example about 100%. The phenyl moieties of the C₁₀-C₁₄ linear alkylphenyl sulfonates each optionally have a biobased content of at least about 50%, preferably at least about 75%, more preferably at least about 95%, for example about 100%.

The mixture of C₁₀-C₁₄ linear alkylphenyl sulfonates comprises less than about 10 wt.%, preferably less than about 5 wt.%, more preferably less than about 3 wt.%, for example about 2 wt.% of linear alkylphenyl sulfonates with branched alkyl groups.

The mixture of C₁₀-C₁₄ linear alkylphenyl sulfonates comprises less than about 5 wt.%, preferably less than about 3 wt.%, more preferably less than about 1 wt.%, for example about 0 wt.% of linear alkylphenyl sulfonates with alkyl groups that have 9 or fewer carbon atoms and alkyl groups that have 15 or more carbon atoms, based on the total weight of the mixture. Linear alkylphenyl sulfonates having alkyl groups with 9 or fewer carbon atoms and 15 or more carbon atoms are undesirable, as previously described.

Further, the mixture of C₁₀-C₁₄ linear alkylphenyl sulfonates comprises less than about 10 wt.%, preferably less than about 7 wt.%, more preferably less than about 5 wt.%, even more preferably less than about 3 wt.%, for example, less than about 1 wt.% of linear C₁₀-C₁₄ alkylphenyl sulfonates having two or more C₁₀-C₁₄ alkyl groups on the phenyl group, based on the total weight of the mixture. Still further, the mixture of C₁₀-C₁₄ linear alkylphenyl sulfonates comprises less than about 10 wt.%, preferably less than about 7 wt.%, more preferably less than about 5 wt.%, even more preferably less than about 3 wt.%, for example, less than about 1 wt.% of C₁₀-C₁₄ linear alkylphenyl sulfonates having an alkyl group comprising a quaternary carbon atom, based on the total weight of the mixture.

Nonlimiting examples of the C₁₀-C₁₄ linear alkylphenyl sulfonates include linear C₁₀-C₁₄ linear alkylphenyl sulfonates, where the alkyl chain can be attached to any position on the benzene ring, as shown below, where M is hydrogen or an metal ion, such as an alkali metal (e.g., sodium, lithium, potassium), an alkaline earth metal (e.g., calcium, magnesium), or the like. 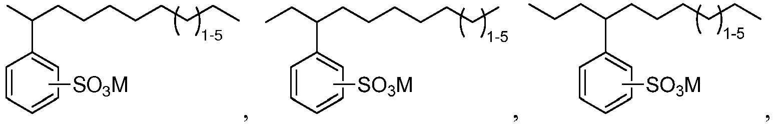 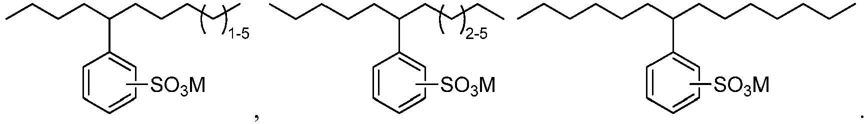 Other nonlimiting examples of C₁₀-C₁₄ linear alkylphenyl sulfonates include 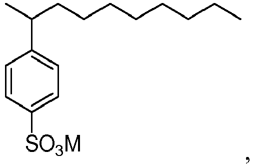 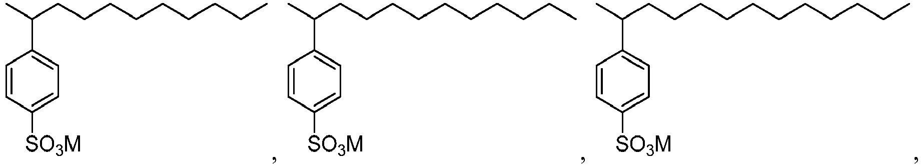 and

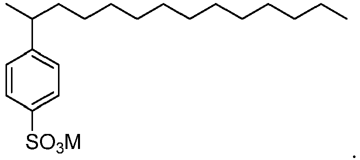

The mixtures of C₁₀-C₁₄ linear alkylphenyl sulfonates are preferably substantially free (i.e., the amounts of said impurity is insufficient to contribute positively or negatively to the effectiveness of the mixture) from dialkyl tetralin impurities. Typically, the mixture comprises less than about 5 wt.%, preferably less than about 1 wt.%, more preferably less than about 0.1 wt.% of dialkyl tetralin, based on the total weight of the mixture.

### Distribution of C₁₀-C₁₄ Alkenes, Linear Alkylbenzenes, and Linear Alkylphenyl Sulfonates

In some specific embodiments, the bio-based mixtures of C₁₀-C₁₄ alkenes, linear alkylbenzenes, and linear alkylphenyl sulfonates of the invention can have a chain length distribution as noted in the below table (in wt%), based upon the total weight of the mixture:

| Olefin Chain length | Avg 11.4 | Avg 11.14 | Avg 11.6 | Avg 11.7 | Avg 12.2 |
|---|---|---|---|---|---|
| 10 | 22 | 32 | 10 | 13 | 9.3 |
| 11 | 31.9 | 33.1 | 37.2 | 30.5 | 21.0 |
| 12 | 29.6 | 25.2 | 32.3 | 31.5 | 25.6 |
| 13 | 16.1 | 9.7 | 19.9 | 24.3 | 30.7 |
| 14 | 0.3 | 0 | 0.7 | 0.7 | 13.5 |

### Blended Embodiments

The renewable C₁₀-C₁₄ linear alkylphenyl sulfonates made by the process of the invention optionally can be blended with petroleum-based C₁₀-C₁₄ linear alkylphenyl sulfonates, and mixtures thereof. In these blended embodiments, blends can be made at any weight ratio of the optionally bio-based to petroleum based compounds, such as, for example, 100:1 to 1:100, 10:90 to 50:50, 51:49 to 92:8.

Further, the particular distributions of bio-based C₁₀-C₁₄ alkenes can be blended with alkenes that have non-traditional distributions. Likewise, the C₁₀-C₁₄ linear alkylbenzenes, and C₁₀-C₁₄ linear alkylphenyl sulfonates of the invention can be blended with alkylbenzenes and alkylphenyl sulfonates having non-traditional alkyl chain distributions to result in compositions having improved performance. For example, a mixture of C₁₀-C₁₄ linear alkylphenyl sulfonate made according to the invention having an alkyl chain distribution as noted in the above table can be spiked into a composition comprising alkylphenyl sulfonates with a non-traditional alkyl chain to result in improved grease cleaning.

### Preparation of the Mixtures of Bio-Based C₁₀-C₁₄ Alkenes

Mixtures of bio-based C₁₀-C₁₄ alkenes having a particular distribution can be prepared using metathesis chemistry. Metathesis involves the reaction of one alkene with another in the presence of a metathesis catalyst to form a new alkene mixture with complete conservation of carbons as illustrated:

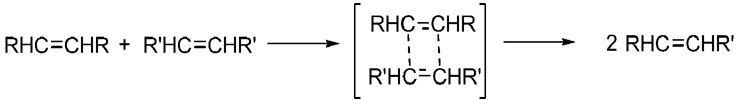

Metathesis chemistry is well known to one skilled in the art (see, e.g., Kirk, "Ruthenium Based Homogeneous Olefin Metathesis," M.S. Dissertation, University of the Free State, South Africa, 2005, Vougioukalakis and Grubbs, Chem. Rev., 110(3):1746-1787 (2010), and U.S. Patent No. 4,943,397).

PCT Application Publication No. WO 2001/02324 discloses a high temperature (e.g., 300-600°C, 1-30 bar or higher) process for the metathesis of Fischer-Tropsch C₅-C₁₅ alkenes (i.e., SASOL process) using a tungsten or molybdenum catalyst (e.g., WO₃ or MoO₃), supported (e.g., by SiO₂, Al₂O₃, ZrO₂, TiO₂, or mixtures thereof) or unsupported, and with or without co-catalysts, to produce C₉-C₁₈ linear and mono-methyl branched alkenes. A method for converting short chain alkenes (e.g., C₄-C₁₀ alkenes) from Fischer-Tropsch derived feedstock to longer chain alkenes (e.g., C₆-C₁₈) using a heterogeneous metal-alkyliene catalyst, such as tungsten, ruthenium (e.g., Grubb's catalyst), osmium, and iridium is disclosed in PCT Application Publication No. WO 2001/046096 and U.S. Patent Application Publication No. 2003/0135080. U.S. Patent No. 5,942,653 discloses the metathesis of alkenes in the presence of a catalyst system comprising silica, alumina, and an alkyl tin compound, but no transition metal. Further descriptions of alkene metatheses using linear or branched alkene starting materials and a tungsten catalyst on a support (e.g., aluminum oxide) can be found in U.S. Patent Application Publication No. 2008/0255328, and U.S. Patent No. 7,635,794.

Metathesis chemistry using fats and/or oils as starting materials is also known in the art. U.S. Patent No. 4,545,941 and U.S. Patent Application Publication No. 2010/0160506, disclose the metathesis of unsaturated triglycerides and alkenes, in the presence of a catalytically effective amount of a metathesis catalyst, to produce modified triglycerides and α-alkenes. U.S. Patent Application Publication No. 2010/0191008, discloses the metathesis of fatty acid esters of oils (e.g., oleic acid, linoleic acid, linolenic acid, vegetable oil, tung oil, meadowfoam oil, coriander oil, camelina oil, jatropha oil, crambe oil, high erucic rapeseed oil, algal oil) and suitable alkenes. U.S. Patent Application Publication No. 2006/0079704, discloses the metathesis of ethylene with unsaturated fats and oils (e.g., oleic sunflower oils, oleic rapeseed oils, and monoalcohol esters thereof) in the presence of a ruthenium metathesis catalyst and at least one non-aqueous ionic liquid.

Ngo et al., JAOCS 83(7):629-634 (2006), describes the solvent-free, self-metathesis of monounsaturated fatty acids of varying purity using the second-generation Grubbs catalyst to form monounsaturated dicarboxylic acids and hydrocarbons in high molecular converstions. Marvey et al., "Ruthenium Carbene Mediated Metathesis of Oleate-Type Fatty Compounds," Int. J. Mol. Sci. 9, 615-625 (2008), discloses the self-metathesis of unsaturated fatty acids and esters, and cross-metathesis of the fatty acids and esters with ethylene using Grubb's catalysts (e.g., RuCl₂(PCy₃)₂(=CHPh), RuCl₂(PCy₃)(H₂IMes)(=CHPh)), SASOL's phoban-indenylidene ruthenium catalyst [(PhobCy)₂Cl₂Ru=C₁₅H₁₀], and Hoveyda-Grubbs catalysts. Further, the metathesis of C₂-C₁₀ alkenes with natural feedstocks, such as natural oils (e.g., vegetable oils, fish oil, animal fat) and derivatives of natural oils, such as fatty acids and fatty acid alkyl esters is described in PCT Application Publication No. WO 2010/062958. U.S. Patent Application No. 2010/0145086, discloses the metathesis of internal alkenes with α-alkenes to form terminal alkenes using a ruthenium catalyst. The internal alkene can include seed oils (e.g., soybean oil, sunflower oil, canola oil, safflower oil, cottonseed oil, castor oil, rapeseed oil, peanut oil, corn oil, olive oil, palm oil, sesame oil, grape seed oil), or compounds that are derived from seed oils. The α-alkene can include 1-propene, 1-butene, 1-pentene, 1-hexene, 1-heptene, 1-octene, and higher alkenes. PCT Patent Application No. WO 2008/046106, discloses the metathesis of terminal alkenes with fats and oils (e.g., soybean oil, sunflower oil, canola oil, safflower oil, cottonseed oil, castor oil, rapeseed oil, peanut oil, corn oil, olive oil, palm oil, sesame oil, grape seed oil) to form linear metathesis products using a ruthenium alkylidene catalyst. PCT Patent Application No. WO 2009/020667, discloses a method for improving catalyst efficiency by chemically treating a natural feedstock before introducing the metathesis catalyst to reduce the amount of catalyst poison.

The mixture of bio-based C₁₀-C₁₄ alkenes, as described herein, using metathesis chemistry mayhave a controlled total carbon atom distribution and a biobased content of at least about 5%, at least about 10%, at least about 20%, at least about 30%, at least about 40%, at least about 50%, at least about 60%, at least about 70%, at least about 80%, at least about 90%, at least about 95%, for example about 100%. This method comprises reacting in the presence of a catalytically effective amount of a metathesis catalyst and under standard metathesis conditions:
(a) a fatty acid, a fatty ester, a fat, an oil, or a mixture thereof; and,
(b) an alkene mixture having a total of 2 to 8 carbon atoms.

The fatty acid, fatty ester, fat, and/or oil has an iodine value of at least about 15, preferably at least about 50, more preferably at least about 180. The iodine value, which can be determined using the AOAC Official Method of Analysis (1984), Chapter 28.023, is the mass of iodine in grams that is consumed by 100 grams of a chemical substance (see, e.g., Pocklington, Pure & Appl. Chem. 62(12):2339-2343 (1990)). The higher the iodine number, the greater the unsaturation in the fatty acid, fatty ester, fat and/or oil. If the iodine number is below about 15, then less of the desired bio-based C₁₀-C₁₄ alkene product is produced. Further, the fatty acid, fatty ester, fat, and/or oil comprises at least about 10 wt.%, preferably at least about 20 wt.% of fatty acids, fatty esters, fats, and/oils that have at least 10 carbon atoms, based on the total weight of the fatty acids, fatty esters, fats, and/or oils.

In any of the methods of making the mixture of C₁₀-C₁₄ alkenes disclosed herein, the mixture can be made in one pot. In these methods, the starting materials are selected to result in alkene products having particular concentrations of particular chain lengths. In some embodiments, the mixture of C₁₀-C₁₄ alkenes disclosed herein is produced by synthesizing separate batches of alkenes having one particular number of total carbon atoms (e.g., a C₁₀ batch, a C₁₁ batch, a C₁₂ batch, a C₁₃ batch, and/or a C₁₄ batch) using alkene metathesis, and then combining the batches to form a mixture having a particular distribution. As a result, one can duplicate the existing commercial olefin mixtures to match the paraffin mixtures derived from petroleum, which become olefin/paraffin mixtures in subsequent processing. This minimizes the need for expensive modifications to existing processing plants around the world and minimizes capital expenses if one wishes to use existing plants to produce bio-based linear alkylbenzenes instead of petroleum-based linear alkylbenzenes.
The fatty acid, fatty acid ester, fat (e.g., animal fat), or oil (e.g., terpenes, monoglycerides, diglycerides, triglycerides, and mixtures thereof) can be derived from renewable resources, such as animals or plants. "Fatty acid" refers to a straight chain monocarboxylic acid having a chain length of 8 to 22 carbon atoms, preferably, 12 to 22 carbon atoms, more preferably 16 to 18 carbon atoms.
"Monoglycerides," "diglycerides," and "triglycerides" refer to mono-, di- and tri- esters, respectively, of (i) glycerol and (ii) the same or mixed fatty acids. Nonlimiting examples of fatty acids include oleic acid, myristoleic acid, palmitoleic acid, sapienic acid, linoleic acid, linolenic acid, arachidonic acid, eicosapentaenoic acid, and docosahexaenoic acid. Nonlimiting examples of monoglycerides include monoglycerides of any of the fatty acids described herein. Nonlimiting examples of diglycerides include diglycerides of any of the fatty acids described herein. Nonlimiting examples of the triglycerides include triglycerides of any of the fatty acids described herein, such as, for example, tall oil, corn oil, soybean oil, sunflower oil, safflower oil, linseed oil, perilla oil, cotton seed oil, tung oil, peanut oil, oiticica oil, hempseed oil, marine oil (e.g., alkali-refined fish oil), dehydrated castor oil, and mixtures thereof.

Preferably, the fatty acid, fatty ester, fat, or oil is selected from or derived from the group consisting of palm oil, kernel oil, coconut oil, rapeseed oil, canola oil, soybean oil, algae oil, cottonseed oil, Jatropha oil, babasu oil, fish oil, linseed oil, tall oil, tallow, poultry fat, camolina, cuphea, a microorganism (e.g., bacteria, yeast, and a mixture thereof), and mixtures thereof. Even more preferably the fatty acid, fatty ester, fat, or oil is selected from or derived from the group consisting of palm oil, rapeseed oil, canola oil, soybean oil, cottonseed oil, jatropha oil, babasu oil, tallow, poultry fat, cuphea, and mixtures thereof. In some embodiments, the fatty acid, fatty ester, fat, or oil is obtained from plants with very high levels of monounsaturated fatty acid, such as from DuPont's PLENISH™ or Monsanto's VISTAGOLD™ high oleic soybean, or the USDA's HA458, HA459 and HA460 high oleic sunflower.

The fatty acids, fatty esters, fats, and oils of the invention can be obtained from their natural sources, as previously described. In some embodiments, the fatty acid, fatty ester, fat, or oil of the invention having a particular chain length also can be produced using engineered oil seed plants. For example, the mid-chain fatty acyl-ACP thioesterase genes, such as from several species in the genus *Cuphea* incuding *procumbens, lutea, hookeriana, hyssopifolia, wrightii* and *inflate,* the Lauraceae family, e.g., the California Bay (*Umbellularia californica*), Pisa (*Actinodophne hookeri*), Sweet Bay (*Laurus nobilis*) and *Cinnamomum camphora* (camphor), and other plant sources, such as *Ulmaceae* (elm), *Myristicaceae, Simarubaceae, Vochysiaceae,* and *Salvadoraceae* can be expressed in oil seed plants, such as Canola, which then accumulate medium chain (e.g., C₁₂, C₁₄) fatty acid containing lipids (see, e.g, U.S. Patent Nos. 5,298,421; 5,304,481; 5,344,771; 5,512,482; and 5,850,022). The fatty acid, fatty ester, fat, or oil of the invention also can be produced by any other method known to one skilled in the art, such as through polyketide synthesis (see e.g., Rawlings, Nat. Prod. Rep. 16:425-484 (1999) and Hranueli et al., Food Technol. Biotechnol 39(3):203-213 (2001)).

The fatty acids, fatty esters, fats, and oils may have been modified from a natural form into an unnatural form (e.g., skeletally isomerized, double-bond isomerized, and/or partially hydrogenated to remove di- and tri-unsaturation) before being subjected to the metathesis reaction.

The alkene having a total of 2 to 8 carbon atoms can include any alkene having 2 to 8 carbon atoms, such as, for example, ethylene, propylene, 1-butene, 2-butene, 1-pentene, 2-pentene, 1-hexene, 2-hexene, 3-hexene, 1-heptene, 2-heptene, 3-heptene, 1-octene, 2-octene, 3-octene, and mixtures thereof. Preferably the alkene having a total of 2 to 8 carbon atoms is selected from the group consisting of ethylene, propylene, a linear butene, a linear pentene, a linear hexene, a linear heptene, a linear octene, and mixtures thereof. More preferably the alkene having a total of 2 to 8 carbon atoms is selected from the group consisting of ethylene, propylene, 1-butene, 2-butene, 1-pentene, 2-pentene, 3-hexene, 3-heptene, and mixtures thereof. In some optional embodiments, the alkene having a total of 2 to 8 carbon is 1-pentene and 2-pentene. In some embodiments the C₂-C₈ alkene is wholly or partially derived from a renewable resource. In alternative embodiments, the C₂-C₈ alkene is not derived from renewable resource (e.g., is petroleum-based).

The mixture of C₁₀-C₁₄ alkenes that results from the metathesis reaction is as described herein. The mixture of C₁₀-C₁₄ alkenes produced from alkene metathesis comprises alkenes that have a biobased content of at least about 5%, at least about 10%, at least about 20%, at least about 30%, at least about 40%, at least about 50%, at least about 60%, at least about 70%, at least about 80%, at least about 90%, at least about 95%, for example about 100%. The alkenes each independently have a total of 10 to 14 carbon atoms. The mixture comprises less than about 10 wt.%, preferably less than about 8 wt.%, more preferably less than about 5 wt.%, for example about 2 wt.% of alkenes with branching. The mixture comprises less than about 5 wt.%, preferably less than about 3 wt.%, more preferably less than about 1 wt.%, for example about 0 wt.% of alkenes that have 9 or fewer carbon atoms and alkenes that have 15 or more carbon atoms, based on the total weight of the mixture. Further, the mixture of C₁₀-C₁₄ alkenes optionally comprises alkanes in an amount less than about 50 wt.%, preferably less than about 25 wt.%, more preferably less than about 5 wt.%, for example, less than about 1 wt.%. Further still, the mixture of C₁₀-C₁₄ alkenes optionally comprises less than about 10 wt.%, preferably less than about 5 wt.%, more preferably less than about 1 wt.% of oxygen-containing compounds (e.g., fatty esters, glycerin).

In some embodiments, the mixture of C₁₀-C₁₄ alkenes produced from alkene metathesis is substantially monounsaturated. In some optional embodiments, the mixture of C₁₀-C₁₄ alkenes is partially hydrogenated to form a mixture of substantially monounsaturated C₁₀-C₁₄ alkenes. Partial hydrogenation can occur by any method known to one skilled in the art. U.S. Patent No. 6,627,778, describes catalysts and reaction conditions that can be used to convert tri-alkenes and di-alkenes into mono-alkenes. This method also can be applied to polyunsaturated fatty acids, fatty esters, fat and oil starting materials to form mono-saturated compounds. For example, the mixture of C₁₀-C₁₄ alkenes can be treated with hydrogen and a catalyst, such as a platinum, palladium, rhodium, ruthenium, or nickel (e.g., Raney nickel, Urushibara nickel) catalyst. Other suitable catalysts for partial hydrogenation are described in U.S. Patent Nos. 4,695,560; 4,523,048; 4,520,214; and 4,761,509 and Chinese Patent No. CN 1032157.

In some embodiments, the partial hydrogenation catalyst can contain about 1.0 wt.% to about 25 wt. % of nickel, about 0.05 wt.% to about 1.5 wt % of sulfur with a support comprising small Al₂O₃ balls made by the oil-drop method. These balls have a pore volume of about 1.44 to about 3.0 cm³/g, a surface area larger than 150 m²/g, have no precious metals, and essentially no halogens, alkali earth metals and alkali metals (e.g., less than about 0.1 wt %). Because the main active element of the catalyst used in this process is nickel, selective hydrogenation is conducted at a temperature greater than about 200°C. Further, the catalyst can be sulfurized to suppress its activity to increase the selectivity of conversion of tri-alkenes and di-alkenes, to mono-alkenes. The step of partial hydrogenation can reduce the amount of dialkenes that are optionally present in the mixture to avoid the formation of tetralins during benzene alkylation, which are not biodegradable.
One can also use high oleoyl content oils. Such oil feedstocks have been demonstrated to provide multiple commercial advantages such as 1) no need for partial hydrogenation to lower the di- and tri-alkene content; 2) increased productivity of desired alkenes in the C₁₀-C₁₄ chain length range, with increased productivity shown as high as 50%, and 3) ready conversion to alkylbenzenes with various alkylation agents already in practice without further modification or formation of substantial undesirable by-products such as indans and tetralins.
Desirable feedstocks of the high oleoyl type are those from soybeans or sunflower oil. High oleoyl containing oils can also be prepared by partial hydrogenation of various oils to reduce the linolinic and linoleic content. However, partial hydrogenation can also increase the level of saturated fatty acids which do not lead to productive olefin formation products via metathesis.

In some optional embodiments, the mixture of C₁₀-C₁₄ alkenes or substantially monounsaturated C₁₀-C₁₄ alkenes is isolated from a crude reaction mixture. Isolation can occur by any method known to one skilled in the art, such as fractional distillation, and simple distillation. Preferably, the mixture of C₁₀-C₁₄ alkenes or substantially monounsaturated C₁₀-C₁₄ alkenes is isolated by simple distillation. In some embodiments, the isolated mixture of C₁₀-C₁₄ alkenes has a purity of at least about 80%, preferably at least about 90%, more preferably at least about 95%, as determined by gas chromatography (GC).

The catalyst used in the metathesis reaction can be any metathesis catalyst or catalyst system useful to catalyze the metathesis reaction of the invention to the desired extent. Any known or future metathesis catalyst can be employed alone, or in combination, with one or more additional catalysts. In some embodiments, the catalyst is quenched and distilled before use. Quenching can be carried out by methyl vinyl ether or removal of the catalyst by absorption onto, e.g., clays. Examples of suitable metathesis catalysts include metal carbene catalysts based on transition metals, such as, for example, ruthenium, chromium, rhenium, tungsten/tin, molybdenum, osmium, titanium, and mixtures thereof. Preferred metathesis catalysts can be based on transition metals selected from the group consisting of a ruthenium catalyst, a molybdenum catalyst, a tungsten/tin catalyst, a rhenium catalyst, a titanium catalyst, and mixtures thereof.

Nonlimiting, specific examples of catalysts appropriate for the production of the mixtures of renewable C₁₀-C₁₄ α-alkenes of the invention include the Tebbe complex, a tungsten dicarbonyl complex (e.g., W(CO)₅CPhOCH₃, W(CO)₅CPh₂) Grubbs first generation catalyst [Ru(Cl)₂(PCy₃)₂CHPh], Grubbs second generation catalyst [Ru(Cl)₂(PCy₃)₂(NHC)CHPh], where NHC is a bulky *N*-heterocyclic carbene ligand H₂IMes, a Schrock carbene complex (e.g., Ta=CH-*t*-Bu(CH₂-*t*-Bu)₃, [W(O)(=CH-*t*-Bu)(PEt₃)₂Cl₂]), or any of the catalysts described in Vougioukalakis and Grubbs, Chem. Rev., 110(3):1746-1787 (2010), and U.S. Patent Application Nos. 2009/0217568 and 2010/0145086. Other examples of suitable catalysts include SASOL's Ru-alkylidene catalyst that contains a phosphorus containing ligand, such as phosphabicylononane, as described in U.S. Patent No. 7,671,224, U.S. Patent Application Publication No. 2008/0221345, and PCT Patent Application Publication No. 2007/010453, examples of which are shown below.

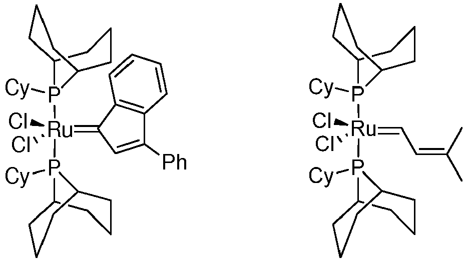

Hoveyda-Grubbs catalysts are also suitable catalysts for the invention, as described in Marvey et al., "Ruthenium Carbene Mediated Meathesis of Oleate-Type Fatty Compounds," Int. J. Mol. Sci. 9, 615-625 (2008), and WO 2010/062958. An example of a Hoveyda-Grubbs catalyst is shown below.

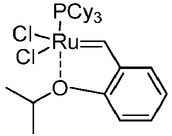

Polymer-bound catalysts, examples of which are described in Buchmeiser, "Polymer-Supported Well-Defined Metathesis Catalysts," Chem. Rev., 109, 303-321, 2009, also can be used for the metathesis reaction of the invention.

In some embodiments, the metathesis reaction is carried out in the presence of a phenolic compound (e.g., phenol, substituted phenol), as described in U.S. Patent Application Publication No. 2006/0211905. The phenolic compound enhances the turnover of the catalyst, which slows down deactivation of the catalyst.

### Metathesis Reaction Conditions

The metathesis reaction can be carried out neat or in an organic solvent. The presence of a solvent improves mixing and, if added to the fatty acid, fatty ester, , fat, and/or oil and partially distilled off before reaction, helps remove traces of water which can poison some metathesis catalysts (e.g., tungsten hexachloride). The more commonly used solvents in metathesis reactions include aliphatic solvents (e.g., saturated hydrocarbons) and aromatic solvents (e.g., benzene, chlorobenzene, and toluene). The aliphatic solvents are preferred over the aromatic solvents because of a reduced tendency to interact with the reactants. In some preferred embodiments, the solvent is a saturated hydrocarbon that boils in the range of about 50°C to about 120°C (e.g., commercial hexane).

In some embodiments, the metathesis reaction is carried out at a temperature of about 35°C to about 260°C, preferably about 50°C to about 120°C. The reaction does not proceed to a noticeable degree at temperatures below about 35°C. The rate of the reaction increases with increasing temperature. Temperatures above about 260°C, however, are undesirable because the starting materials begin to degrade.

### Sources of Bio-Based Alkenes for the Metathesis Reaction

Bio-based alkenes can be produced from any renewable source, such as the decarboxylation of natural fats and oils under low or no hydrogen conditions (e.g., C₈-C₂₂ fatty acids, monoglycerides and diglycerides of C₈-C₂₂ fatty acids, C₁-C₄ alkyl esters of C₈-C₂₂ fatty acids) using an activated acidic catalyst free of Group VIII metals (e.g., Sn/Pt), as described in PCT Patent Application No. WO 2007/136873. For example, alkenes produced from seed oil derived soy fatty acid methyl esters using 1-propene or 1-butene would result in 9.8% of 1-decene, 5.4% of 2-undecene, 17.5% of methyl 9-decenoate, and 13.9% of methyl 9-undecenoate, or 10.5% of 1-decene, 8.2% of 3-dodecene, 19.6% of methyl 9-decenoate, and 14.6% of methyl 9-dodecenoate , as described in PCT Application Publication No 2008/046106. PCT Application No. WO 2008/046106, also describes the metathesis of 1-butene with soy oil derived fatty acid methyl esters.

Short chain bio-based C₂-C₄ alkenes can be used as co-reactants in the metathesis reactions to provide longer chain alkene products that are 100% biobased. These bio-based short chain alkenes can be produced from plant biomass, as described in Paushkin, et al., Chemistry and Technology of Fuels and Oils 30(4-5):249-252 (1994) and Khokhlachev et al., Khimiya i Tekhnologiaya Topliv i Masel 6:3-5 (1994). In this process, plant raw material undergoes steam gasification at 1000-1200°C, as shown in the below scheme. The products of gasification containing carbon monoxide and hydrogen are then reacted with a Co catalyst (200°C, 1 MPa) to form liquid hydrocarbons. These liquid hydrocarbons are purified and subjected to pyrolysis over a KVO₄ catalyst at 790°C to form the C₂-C₄ alkenes.

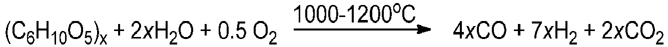

Bio-based C₂-C₈ alkenes also can be produced using the SASOL process. In this process, bio-based 1-butene is isomerized *in situ* to 2-butene, which undergoes metathesis with the remaining 1-butene to produce propene and 2-pentene. Self-metathesis of the 1-butene produces 3-hexene and ethene. The ethene can undergo metathesis with the 2-butene and 2-pentene to form additional propene.

Another source of bio-based C₄-C₈ alkenes is lignocellulosic wastes. Alkenes can be produced from this waste by subjecting the waste to acid hydrolysis to form levulinic acid. The levulinic acid is then catalytically upgraded to 5-nonane with the intermediate formation of y-valerolactone (GVL) (Bond et al., Science 327:110-114 (2010) and Bond et al., Langmuir 26(21):16291-16298 (2010)). Specifically, the GVL is produced by the hydrogenation of levulinic acid. GVL can be processed with a combined decarboxylation and oligomerization strategy to form alkenes. In this process, GVL undergoes a ring opening to produce an isomeric mixture of unsaturated pentenoic acids, which then undergo decarboxylation to produce butane isomers and a stoichiometric quantity of carbon dioxide. This reaction can be carried out over a solid acid catalyst, SiO₂/Al₂O₃, in the presence of water and at a pressure of ambient up to 36 bar. A separation step occurs where water is condensed to the liquid state and the butane undergoes acid-catalyzed oligomerization to higher molecular weight alkenes. This oligomerization process is favored at elevated pressures and can be tuned to yield alkenes with a targeted range of molecular weights and varied degrees of branching.

The GVL process for the preparation of alkenes is advantageous because it provides a mixture of alkenes that is not random. In addition, it produces a carbon dioxide stream at elevated pressure (e.g., 36 bar), which is appropriate for sequestration, conversion to methanol upon reaction with a renewable source of hydrogen, or copolymerization with epoxides to yield polycarbonates. By contrast, the production of carbon dioxide during fermentation of glucose to ethanol is carried out at atmospheric pressure in the presence of air.

Bio-based C₂-C₈ alkenes also can be produced from sugars. For example, bio-ethylene and bio-propylene can be formed from the dehydration of bio-ethanol and bio-propanol, respectively. Bio-based ethanol and bio-based propanol can be derived from, for example, (i) the fermentation of sugar from sugar cane, sugar beet, or sorghum; (ii) the saccharification of starch from maize, wheat, or manioc; and (iii) the hydrolysis of cellulosic materials. U.S. Patent Application Publication No. 2005/0272134, describes the fermentation of sugars to form alcohols and acids.

Suitable sugars used to form ethanol and propanol include monosaccharides, disaccharides, trisaccharides, and oligosaccharides. Sugars, such as sucrose, glucose, fructose, and maltose, are readily produced from renewable resources, such as sugar cane and sugar beets. Sugars also can be derived (e.g., via enzymatic cleavage) from other agricultural products (i.e., renewable resources resulting from the cultivation of land or the husbandry of animals). For example, glucose can be prepared on a commercial scale by enzymatic hydrolysis of corn starch. Other common agricultural crops that can be used as the base starch for conversion into glucose include wheat, buckwheat, arracaha, potato, barley, kudzu, cassava, sorghum, sweet potato, yam, arrowroot, sago, and other like starchy fruit, seeds, or tubers. The sugars produced by these renewable resources (e.g., corn starch from corn) can be used to produce alcohols, such as propanol, ethanol, and methanol. For example, corn starch can be enzymatically hydrolyzed to yield glucose and/or other sugars. The resultant sugars can be converted into ethanol and propanol by fermentation.

Bio-propanol also can be derived from bio-ethylene. In this pathway, bio-ethylene is converted into propionaldehyde by hydroformylation using carbon monoxide and hydrogen in the presence of a catalyst, such as cobalt octacarbonyl or a rhodium complex. Hydrogenation of the propionaldehyde in the presence of a catalyst, such as sodium borohydride and lithium aluminum hydride, yields propan-1-ol, which can be dehydrated in an acid catalyzed reaction to yield propylene, as described in U.S. Patent Application Publication No. 2007/0219521.

In some embodiments, bio-ethanol can be dehydrated to ethylene then oligomerized via the Shell Higher Olefin Process (SHOP®, Shell Chemicals), as described in Scheibel, Journal of Surfactants and Detergents, "The Evolution of Anionic Surfactant Technology to Meet the Requirements of the Laundry Detergent Industry", 7(4):319-328 (2004), to form bio-based alpha olefins with an even chain length. These can be further processed to form even and odd internal alkenes. These types of bio-based alkenes can then be metathesized to produced mixed bio-internal alkenes with even and odd chains.

Bio-based ethylene and bio-based propylene also can be produced from biomass waste (e.g., wood, agricultural waste, municipal waste) using the methanol-to-oil (MTO) process, as described in a PowerPoint presentation, "Biomass Waste to Olefin Technology," MIT (2005) by Chiang. In the pretreatment step of this process, the biomass waste is chipped or grinded to a proper size and dried. The resulting product is subjected to steam, oxygen, and heat in a gasification step, to result in hydrogen, water, carbon monoxide, carbon dioxide, methane, ethane, and other by-products, which are subsequently removed. The methane and ethane are converted to carbon monoxide and hydrogen using a nickel-based catalyst. The amount of carbon monoxide is adjusted using the water-gas shift reaction (H₂O + CO → CO₂ + H₂O), and the amount of carbon dioxide is adjusted using chemical absorption to result in a H₂:CO ratio of 2:1, with relatively small amounts of carbon dioxide. The hydrogen then reacts with carbon monoxide and carbon dioxide to form methanol. The methanol is fed into a reactor, a catalyst is added, product gas (e.g., methane, ethane, propane, carbon dioxide, water) is cooled and some water is condensed. The carbon dioxide is removed, as well as the remaining water. Ethylene and propylene are recovered. Three tones of methanol is required to produce each tonne of ethylene/propylene. The process can yield 0.8 to 1.3 tonnes of propylene per ton of ethylene.

Other sources of bio-based ethylene are as follows. In higher plants, bio-ethylene can be produced via amino-cyclopropane-1-carboxylic acid (ACC) according to the ACC pathway. In microorganisms, bio-ethylene can be synthesized according to the KMBA pathway. In fungi *Penicillium cyclopium, P. digitatum, F. oxisporum* and in bacteria *P. syringae,* bio-ethylene can be produced using 2-oxoglutaric acid as a precursor and the multifunction enzyme termed "ethylene-forming enzyme" (EFE).

Although less preferred, the alkene starting materials of the invention can be prepared from the partial or complete dehydrogenation of bio-based paraffin feedstock using any method known to one skilled in the art. In general, dehydrogenation of the paraffin can be accomplished using any of the well-known dehydrogenation catalyst systems or "conventional dehydrogenation catalysts" including those described in the Surfactant Science Series references previously cited as well as in "Detergent Manufacture Including Zeolite Builders and Other New Materials", Ed. Sittig, Noyes Data Corp., New Jersey, 1979, and other dehydrogenation catalyst systems, for example those commercially available though UOP Corp. Dehydrogenation can be conducted in presence of hydrogen gas and, commonly, a precious metal catalyst. Alternatively, non-hydrogen, precious-metal free dehydrogenation systems such as a zeolite/air system can be used. As is well known, dehydrogenation can be complete or partial, more typically partial. When dehydrogenation is partial, a mixture of alkenes and unreacted paraffin results. Such mixture is a suitable feed for the alkylation step of the invention, as long as the mixture includes less than about 50 wt.%, preferably less than about 25 wt.%, more preferably less than about 5 wt.%, for example, less than about 1 wt.% of paraffins.

Bio-based short chain alkenes also can be produced from alkanes that are produced from cellulose, as described in Serrano-Ruiz et al., Applied Catalysis B: Environmental 100(1-2):184-189 (2010). In this process, solid cellulose is deconstructed to produce glucose using aqueous sulfuric acid. The glucose is subsequently dehydrated under acidic conditions to generate an equimolar mixture of levulinic acid and formic acid. The formic acid is decomposed to hydrogen and carbon dioxide, and the hydrogen is used to reduce the levulinic acid to GVL over a Ru/C catalyst. The GVL product is more hydrophobic than levulinic acid, thereby enabling selected separation of sulfuric acid from GVL, and allowing most of the acid to be recycled back to the cellulose deconstruction reactor. An aqueous solution of GVL containing smaller amounts of sulfuric acid is then passed over a sulfur-tolerant niobia-supported palladium catalyst in the presence of hydrogen to produce pentanoic acid, followed by conversion to 5-nonanone by ketonization over a ceria-zirconia catalyst. The hydrophobic stream of 5-nonane can be further processed to liquid alkanes with controlled structures by means of well-established hydrogenation, dehydration and/or isomerization reactions. The alkanes can be converted to alkenes by any method known to one skilled in the art.

Another source of alkanes that can be converted to the alkenes of the invention is described in U.S. Patent Application Publication No. 2009/0124839. In this process, a feedstock solution comprising a carbohydrate is dehydrated in the presence of an acid to yield at least one furan derivative compound. The furan derivative compound is subjected to at least one self-aldol condensation reaction or a crossed-aldol condensation reaction with another carbonyl compound to yield a beta-hydroxy carbonyl compound and/or an alpha-beta unsaturated carbonyl compound. The beta-hydroxy carbonyl and/or alpha-beta unsaturated compounds are then hydrogenated to yield a saturated or partially saturated compound, which undergoes hydrodeoxygenation (e.g., dehydration and hydrogenation) to yield a composition comprising C₈-C₁₅ alkanes.

Other sources for the production of bio-based alkanes are disclosed in Lennen et al., Biotechnology and Bioengineering 106(2):193-202 (2010), West et al., Catalysis Communications 10(13):1743-1746 (2009), Kunkes et al., Science 322(5900):417-412 (2008), West et al., ChemSusChem 1(5):417-424 (2008), Huber et al., Angewandte Chemie, International Edition 43(12):1549-1551 (2004), and Huber et al., Science 308(5727:1446-1450 (2005).

### Metabolically Engineered Microorganisms

The mixtures of bio-based C₁₀-C₁₄ linear alkenes having a particular distribution also can be prepared using metabolically engineered organisms. The preparation of fatty alcohols, fatty aldehydes, fatty acids, and derivatives thereof from genetically-modified cells and microrganisms for use in applications such as biofuels, polymers, surfactants, lubricating oil additives, and intermediates for the production of derivatives such as acrylates used in paints, coatings, and adhesive applications, is described in U.S. Patent Application Publication Nos. 2010/0105955 and 2010/0105963; and International Patent Application Publication Nos. WO 2007/136752, WO 2008/119082, and WO 2009/111672.

For example, Ladygina et al., Process Biochemistry 41:1001-1014 (2006) discloses the microbial production of intracellular, straight chain and branched chain hydrocarbons having different chain lengths from different microorganisms (e.g., cyanobacteria, aerobic bacteria, anaerobic bacteria, yeasts, mycelia fungi). Alvarez et al., Appl. Microbiol. Biotechnol 60:367-376 (2002), discloses the biosynthesis of high amounts of triacylglycerols, which can be converted into fatty acids, in bacteria (e.g., *Mycobacterium, Streptomyces, Rhodococcus, Nocardia*). The compositions and structures of the triacylglycerols vary depending on the microorganism and the carbon source. Magnuson et al., Microbiol. Mol. Biol. Rev. 57(3):522-542 (1993), discloses the regulation of fatty acid biosynthesis in *E. coli* (e.g., the level of expression of the fabA and fabB genes appear to establish a basal ratio of unsaturated to saturated fatty acid synthesis in the absence of thermal regulation).

U.S. Patent Application Publication No. 2009/0275097 and PCT Patent Application Publication No. WO 2009/111672, disclose routes to producing long chain primary alcohols. In these routes, acyl-CoAs are produced and then converted to the corresponding aldehydes using fatty acyl Co-A reductase. The aldehydes are reduced to long chain primary alcohols. Specifically, a non-naturally occurring microbial organism comprising a malonyl-CoA-independent fatty acid synthesis pathway and an acyl-reduction pathway is cultured. The malonyl-CoA-independent fatty acid synthesis pathway comprises exogenous nucleic acids encoding ketoacyl-CoA acyltransferase or ketoacyl CoA thioase, 3-hydroxyacyl-CoA dehydrogenase, enoyl-CoA hydratase and enoyl-CoA reductase. The acyl-reduction pathway comprises one or more exogenous nucleic acids encoding an acyl-CoA reductase and an alcohol dehydrogenase.

U.S. Patent Application Publication No. 2009/061493, discloses methods of cultivating microorganisms (e.g., microalgae cell, oleaginous yeast, fungus) containing an exogenous genes that codes a protein selected from the group consisting of a lipase, sucrose transporter, sucrose invertase, fructokinase, polysaccharide-degrading enzyme, a fatty acyl-ACP thioesterase, a fatty acyl-CoA/aldehyde reductase, a fatty aldehyde decarbonylase, and an acyl carrier protein (ACP) for the production of lipids, fatty acids, fatty esters, aldehydes, alcohols, and straight chain alkanes.

PCT Application Publication Nos. WO 2009/085278, WO 2009/140695 and WO 2009/140696, describe methods of producing hydrocarbons, fatty acids, fatty aldehydes, fatty alcohols, fatty esters, acyl-CoAs, acyl-ACPs, and/or fatty acid derivatives by engineering microorganisms. The hydrocarbon can be an alkane or an alkene. The alkene can be a terminal C₃-C₂₅ alkene, straight chain or branched chain, and/or cyclic. The alkane can be a C₃-C₂₅ alkane, straight chain or branched chain, and/or cyclic. The fatty acid, fatty aldehyde, fatty alcohol, and/or fatty ester, can comprise C₆-C₂₆ carbon atoms, and can be unsaturated or saturated, straight chain or branched chain, and can include a cyclic moiety. In the '278 application, the alkanes and alkenes are produced by the reduction of fatty acyl-ACP to aldehydes, followed by decarbonylation. In the '696 application, alkanes and alkenes are produced by the direct decarboxylation of fatty acids.

Other references that disclose the production of fatty acids and derivatives thereof using engineering microorganisms include PCT Application Publication Nos. WO 2010/075483, WO 2010/062480, WO 2010/042664, WO 2010/022090, WO 2010/021711, WO 2009/042950, WO 2009/009391, WO 2008/147781, WO 2008/119082, WO 2008/113041, WO 2008/100251, WO 2007/136762, and U.S. Patent Application Publication Nos. 2010/0221798, 2010/0199548, 2010/0170826, 2010/0105963, 2010/0105955, 2010/0071259, 2008/0293060.

The mixtures of C₁₀-C₁₄ alkenes having a particular distribution can be produced using the previously described methods that utilize engineered microorganisms. In these methods, various host cells can be used, as previously described. In some embodiments, exogenous acyl-ACP reductase and exogenouse decarbonylase are expressed in a microorganism (e.g., *E. coli*) to generate alkanes and/or alkenes with particular chain lengths via the fatty acid synthesis pathway, as previously described. In alternative embodiments, fatty acids can be fermented in two different organisms with their maximum production each at a particular chain length. The products then can be combined.

Using microorganisms to produce the mixtures of C₁₀-C₁₄ alkenes having a particular distribution is advantageous. These methods allow complete tunablility to produce mixtures of alkenes having different distributions. They minimizes the requirement of chemically oxidizing paraffins because the product that is produced as a higher alkene to paraffin ratio than traditional processes. They allow the production of linear, branched, or a mixture of linear and branched alkenes. Further, a variety of gene sources can be used to produce the fatty acid precursors and to convert fatty acids, fatty acyl-ACP, fatty acyl-CoAs into alkanes and alkenes. Further still the process uses low cost, renewable feedstocks (i.e., sugar) instead of petroleum-based feedstocks.

The fatty acids or esters or triglycerides prepared either from such microorganisms or from standard plant based fats and oils or fatty acids can be decarboxylated to alkenes as described in PCT Patent Application No. WO 2007/136873. Fatty alcohols derived from microbial production either directly via bioengineering or via microbial production of the methyl ester or fatty acid can be converted into fatty alcohols by standard reduction practices in the industry. Plant derived fats and oils can also be transesterified and reduced to fatty alcohols. Both types of fatty alcohols can also become alkene feedstock for production of alkylbenzenes as described herein by means of industry standard practices for alcohol dehydration such as practiced by WO09905084A1 or other industrially relevant processes used for alcohol dehydration. Furthermore, the fatty alcohols can also be converted directly to a bio LAB without the need for dehydration using as described in WO09905084A1.

### Alkylation of Benzene with the Mixture of Bio-Based C₁₀-C₁₄ Alkenes

The mixture of bio-based C₁₀-C₁₄ alkenes produced by dehydrating a C₁₀-C₁₄ alcohol mixture can be used to alkylate benzene to form a mixture of C₁₀-C₁₄ linear alkylbenzenes having particular alkyl chain distributions, as described herein.

The benzene used for alkylation can be derived from a renewable resource, a petroleum resource, or a mixture thereof. In some optional embodiments, benzene has a biobased content of at least about 50%, more preferably at least about 75%, even more preferably at least about 95%, for example about 100%, as determined by ASTM D6866. Bio-based benzene can be produced by the metathesis of any C18-3 component of oil/fat (e.g., linoleic type) to generate 1,4,7-decatriene. The 1,4,7-decatriene is cyclized *in situ,* in the presence of a metathesis catalyst, to form 1,3-cyclohexadiene, and then the cyclohexadiene is dehydrogenated to form bio-benzene. Bio-based benzene also can be produced from the hydrocracking of lignin, which results in bio-based benzene and phenol. The bio-based phenol can be subsequently dehydroxylated to produce the bio-based benzene, as described in U.S. Patent No. 4,420,644. Bio-based benzene can also be produced by treating carbohydrates in an aqueous phase with catalytic chemistry to reduce the oxygen content; this aqueous mixture can then be treated with conventional petrochemical catalysts to produce mixtures of benzene, toluene and xylenes. The bio-based benzene can be separated by conventional processes and used in the alkylation process. The process to produce this catalytically derived benzene is described in U.S. Patent No. 7,977,517.

Alkylation of benzene by the mixture of bio-based C₁₀-C₁₄ alkenes can be accomplished by any method known to one skilled in the art, see, e.g., U.S. Patent Nos. 6,583,096, 6,514,926, and PCT Patent Application Publication No. WO 2009/079213. For example, alkylation can be performed at a temperature of about 125°C to about 230°C, preferably about 175°C to about 215°C, and at a pressure of about 3.4 barg to about 70 barg (about 50 psig to about 1000 psig), preferably about 7 barg to about 17 barg (about 100 psig to about 250 psig). The reaction time for this alkylation can vary; however, it is preferred that the reaction is about 0.01 hour to about 18 hours, more preferably, as rapidly as possible, more typically about 0.1 hour to about 5 hours, or about 0.1 hour to about 3 hours.

Generally, it is preferable to couple together the use of relatively low temperatures (e.g., about 175°C to about 215°C) with reaction times of medium duration (e.g., 1 hour to about 8 hours) in the above-indicated ranges. Moreover, it is contemplated that the alkylation of the benzene be "staged" so that two or more reactors operating under different conditions in the defined ranges may be useful.

In some embodiments, the C₁₀-C₁₄ linear alkylbenzenes of the invention can be produced directly from fatty acids, fatty esters, fats, and/or oils by combining the fatty acids, fatty esters, fats, and/or oils with benzene in the presence of a good decarboxylation catalyst/weak alkylation catalyst at 300-400°C and simulataneously decarboxylating to alkenes and alkylating the aromatic mixture, as described in U.S. Patent No. 7,683,224.

### Alkylation Catalysts

Alkylation of benzene can be carried out using mineral acids (e.g., solid phosphoric acid) and Friedel-Crafts catalysts (e.g, AlCl₃ HCl). Benzene can be alkylated with linear alkenes using DETAL® process catalysts, HF, aluminum chloride, HF on zeolites, fluoridated zeolites, non-acidic calcium mordenite, and amorphous silica/aluminua. Such process that use these catalysts include the DETAL® process of UOP and CEPSA (Petresa) and processes described in U.S. Patent Nos. 6,602,840; 5,344,997; 5,196,574; 5,334,793; 5,245,094. Optionally, depending on feedstock and the precise sequence of steps used, the present process can include distillation of the alkylbenzenes to remove impurities and by-products, such as unreacted starting materials, paraffins, and excesses of benzene. Any conventional distillation apparatus can be used. The general practice for distillation is similar to that used for distillation of commercial linear alkylbenzenes (LAB), and suitable distillation steps are described in Surfactant Science Series, Marcel Dekker, New York, 1996, including in particular Chapter 2 entitled "Alkylarylsulfonates: History, Manufacture, Analysis and Environmental Properties", pages 39-108 which includes 297 literature references.

The mixture of bio-based C₁₀-C₁₄ linear alkylbenzenes having particular chain length distributions can be made in one pot. In these methods, the alkene reactants are selected to result in linear alkylbenzene products having particular alkyl chain length distribution. In some embodiments, the mixture of C₁₀-C₁₄ linear alkylbenzenes having particular chain length distributions is made by synthesizing separate batches of linear alkylbenzenes, each having alkyl chains with a particular number of total carbon atoms (e.g., a C₁₀ batch, a C₁₁ batch, a C₁₂ batch, a C₁₃ batch, and a C₁₄ batch), and then combining the batches in particular concentrations to form a mixture having a particular alkyl chain distribution.

### Sulfonation of Linear Alkylbenzene

Sulfonation of the linear alkylbenzenes can be accomplished using any sulfonation system, including those described in "Detergent Manufacture Including Zeolite Builders and Other New Materials," Ed. M. Sittig, Noyes Data Corporation, 1979, as well as in the hereinabove-referenced Surfactant Science Series review. Common sulfonation systems include sulfuric acid, chlorosulfonic acid, oleum, sulfur trioxide with and without air. Sulfur trioxide/air is especially preferred, and details of this process are provided in U.S. Patent No. 3,427,342, and de Groot, "Sulfonation Technology in the Detergent Industry" Kluwer Academic Publishers, Boston, 1991.

Any convenient workup steps may be used in the present process. In some embodiments, the product is neutralized after sulfonation using any suitable alkali metal (e.g., sodium, potassium, ammonium, magnesium substituted ammonium alkalis, and mixtures thereof). Potassium can assist solubility, magnesium can promote soft water performance and substituted ammonium can be helpful for formulating specialty variations of the instant surfactants. Sodium-form alkali, such as sodium hydroxide, is most commonly used. In some preferred embodiments, the alkali metals are selected from the group consisting of sodium hydroxide, potassium hydroxide, ammonium hydroxide, sodium carbonate, potassium carbonate, and mixtures thereof. If the alkylphenyl sulfonate is to be mixed with cleaning components, it can be added in the acid form directly to the cleaning composition and then neutralized.

The mixture of C₁₀-C₁₄ linear alkylphenyl sulfonates having particular chain length distributions can be made in one pot. In these methods, the linear alkylbenzenes that are being sulfonated are selected to result in alkylphenyl sulfonate products having particular alkyl chain distributions as described herein. In some embodiments, the mixture of C₁₀-C₁₄ linear alkylphenyl sulfonates having particular chain length distributions is made by synthesizing separate batches of linear alkylphenyl sulfonates, each having a particular number of total carbon atoms on its alkyl chain (e.g., a C₁₀ batch, a C₁₁ batch, a C₁₂ batch, a C₁₃ batch, and a C₁₄ batch), and then combining the batches in particular concentrations to form a mixture of alkylphenyl sulfonates having a particular alkyl chain distribution.

### Blended Embodiments

Prior to the sulfonation step, the bio-based C₁₀-C₁₄ linear alkylbenzene of the invention can blended with petroleum-based linear alkylbenzene. Further, in any step subsequent to said sulfonation step, the bio-based C₁₀-C₁₄ linear alkylphenyl sulfonate of the invention (acid-form or neutralized-form) can be blended with a petroleum-derived linear alkylphenyl sulfonate. In these blended embodiments, blends can be made at a weight ratio of the bio-based compounds to the petroleum-based compounds, or their derivatives, of 100:1 to 1:100, 10:90 to 50:50, or 51:49 to 92:8.

### Assessment of the Bio-Based Content of the Materials

A suitable method to assess materials derived from renewable resources is through ASTM D6866, which allows the determination of the biobased content of materials using radiocarbon analysis by accelerator mass spectrometry, liquid scintillation counting, and isotope mass spectrometry. When nitrogen in the atmosphere is struck by an ultraviolet light produced neutron, it loses a proton and forms carbon that has a molecular weight of 14, which is radioactive. This ¹⁴C is immediately oxidized into carbon dioxide, which represents a small, but measurable fraction of atmospheric carbon. Atmospheric carbon dioxide is cycled by green plants to make organic molecules during the process known as photosynthesis. The cycle is completed when the green plants or other forms of life metabolize the organic molecules producing carbon dioxide, which causes the release of carbon dioxide back to the atmosphere. Virtually all forms of life on Earth depend on this green plant production of organic molecules to produce the chemical energy that facilitates growth and reproduction. Therefore, the ¹⁴C that exists in the atmosphere becomes part of all life forms and their biological products. These renewably based organic molecules that biodegrade to carbon dioxide do not contribute to global warming because no net increase of carbon is emitted to the atmosphere. In contrast, fossil fuel-based carbon does not have the signature radiocarbon ratio of atmospheric carbon dioxide. *See* WO 2009/155086.

The application of ASTM D6866 to derive a "biobased content" is built on the same concepts as radiocarbon dating, but without use of the age equations. The analysis is performed by deriving a ratio of the amount of radiocarbon (¹⁴C) in an unknown sample to that of a modern reference standard. The ratio is reported as a percentage with the units "pMC" (percent modern carbon). If the material being analyzed is a mixture of present day radiocarbon and fossil carbon (containing no radiocarbon), then the pMC value obtained correlates directly to the amount of biomass material present in the sample.

The modern reference standard used in radiocarbon dating is a NIST (National Institute of Standards and Technology) standard with a known radiocarbon content equivalent approximately to the year AD 1950. The year AD 1950 was chosen because it represented a time prior to thermo-nuclear weapons testing, which introduced large amounts of excess radiocarbon into the atmosphere with each explosion (termed "bomb carbon"). The AD 1950 reference represents 100 pMC.

"Bomb carbon" in the atmosphere reached almost twice normal levels in 1963 at the peak of testing and prior to the treaty halting the testing. Its distribution within the atmosphere has been approximated since its appearance, showing values that are greater than 100 pMC for plants and animals living since AD 1950. The distribution of bomb carbon has gradually decreased over time, with today's value being near 107.5 pMC. As a result, a fresh biomass material, such as corn, could result in a radiocarbon signature near 107.5 pMC.

Petroleum-based carbon does not have the signature radiocarbon ratio of atmospheric carbon dioxide. Research has noted that fossil fuels and petrochemicals have less than about 1 pMC, and typically less than about 0.1 pMC, for example, less than about 0.03 pMC. However, compounds derived entirely from renewable resources have at least about 95 percent modern carbon (pMC), preferably at least about 99 pMC, for example, about 100 pMC.

Combining fossil carbon with present day carbon into a material will result in a dilution of the present day pMC content. By presuming that 107.5 pMC represents present day biomass materials and 0 pMC represents petroleum derivatives, the measured pMC value for that material will reflect the proportions of the two component types. A material derived 100% from present day soybeans would give a radiocarbon signature near 107.5 pMC. If that material was diluted with 50% petroleum derivatives, it would give a radiocarbon signature near 54 pMC.

A biobased content result is derived by assigning 100% equal to 107.5 pMC and 0% equal to 0 pMC. In this regard, a sample measuring 99 pMC will give an equivalent biobased content result of 93%.

Assessment of the materials described herein were done in accordance with ASTM D6866, particularly with Method B. The mean values quoted in this report encompasses an absolute range of 6% (plus and minus 3% on either side of the biobased content value) to account for variations in end-component radiocarbon signatures. It is presumed that all materials are present day or fossil in origin and that the desired result is the amount of biobased component "present" in the material, not the amount of biobased material "used" in the manufacturing process.

Other techniques for assessing the biobased content of materials are described in U.S. Patent Nos. 3,885,155, 4,427,884, 4,973,841, 5,438,194, and 5,661,299, and WO 2009/155086.

### Determination of Alkene and Alkyl Chain Distribution

The C₁₀-C₁₄ alkenes, C₁₀-C₁₄ linear alkylbenzenes, and C₁₀-C₁₄ linear alkylphenyl sulfonates can be characterized using gas chromatography and NMR, as described in PCT Application No. WO 2008/046106, for example, on pages 42-45.

For example, analytical analysis of the mixtures of the invention can be performed using gas chromatography. A calibration solution is prepared by weighing 5 grams of pure hexadecane to the nearest 0.0001 g and adding it to a 100 mL volumetric flask. The flask is filled to volume with methylene chloride, stoppered, and mixed well. A sample solution is prepared in the following way. The mixture of the invention is passed through a PTFE syringe filter (0.45 µm) and a 2 mL GC vial is tared. 50 µL of the mixture is dispensed into the GC vial using a micro-pipette and the vial is weighed. 1000 µL of the calibration solution is added to the GC vial and the weight is recorded to the nearest 0.1 mg. The vial is crimp-sealed and the contents of the vial are shaken. The sample is injected into a GC that has the following parameters.

### Fast GC Method Instrument Operation

**Column:** Restek RTX-5 (10244) 105m x 0.25mm x 0.50um df
**Oven:** Maximum temp.: 330°C
Total run time: 35 min Rate: 5.0°C/min
Initial temp: 180°C Final temp: 320°C
Initial time: 0.0 min Final time: 7.0 min
Inlet:
   Mode: Split
   Split Ratio: 50:1
   Inlet temp: 300°C
   Carrier gas: He
   Linear velocity: 20 cm/sec.
Injector:
   Injection volume: 0.2 µL Solvent A&B: DCM (CH₂Cl₂)
   Sample washes: 3 Solvent washes (A): 3
   Sample pumps: 5 Solvent washes (B): 3
Detector (FID):
   Temp. 320°C
   Hydrogen flow: 40 mL/min
   Air flow: 450 mL/min
   Makeup gas: N₂
   Makeup flow: 45 mL/min

### Commercial Uses

The mixtures of bio-based C₁₀-C₁₄ linear alkylphenyl sulfonates of the invention that have particular distributions can be included in consumer product cleaning or personal care compositions for cost and environmental benefits. Thus, in another aspect, the invention relates to a composition comprising about 0.001 wt.% to about 99.999 wt.%, preferably about 0.1 wt.% to about 80 wt.% of the mixture of renewable C₁₀-C₁₄ alkylphenyl sulfonates of the invention, as previously described, and about 0.001 wt.% to about 99.999 wt.% of one or more additional cleaning components or about 0.001 wt.% to about 99.999 wt.% of one or more additional personal care components.

In some alternative embodiments, the mixture made according to the invention comprises bio-based linear alkylphenyl sulfonates primarily having alkyl groups with lower levels of C10 and greater proportions of C11, C12, and C14. In these embodiments, linear alkylphenyl sulfonates having alkyl groups with 13 or 14 carbon atoms are present in an amount of no more than about 30 wt.%, preferably no more than about 20 wt.%, more preferably no more than about 10 wt.%. These represent the longer chain averages marketed for industrial use, such as C12.3 and C12.5 materials. This mixture of linear alkylphenyl sulfonates primarily having C₁₀-C₁₂ alkyl groups is useful for, for example, laundry detergents. In other alternative embodiments, the mixture comprises bio-based linear alkylphenyl sulfonates primarily having alkyl groups with 10 and/or 11 carbon atoms. In these embodiments, linear alkylphenyl sulfonates having alkyl groups with 12 carbon atoms are present in an amount of no more than about 30 wt.%, preferably no more than about 20 wt.%, more preferably no more than about 10 wt.%, and alkylphenyl sulfonates having alkyl groups with 13 or 14 carbon atom are present in an amount of no more than about 5 wt.%. This mixture of linear alkylphenyl sulfonates having primarily C₁₀-C₁₁ alkyl groups is useful for, for example, increasing the sudsing of diswashing liquids.

### Consumer Product Cleaning Compositions

Consumer product cleaning compositions are described in the "Surfactant Science Series", Marcel Dekker, New York, Volumes 1-67 and higher. In particular, liquid compositions are described in detail in Volume 67, "Liquid Detergents," Ed. Kuo-Yann Lai, 1997, ISBN 0-8247-9391-9. More classical formulations, especially granular type formulations, are described in "Detergent Manufacture including Zeolite Builders and Other New Materials", Ed. M. Sittig, Noyes Data Corporation, 1979, incorporated herein by reference. See also Kirk Othmer's Encyclopedia of Chemical Technology. Nonlimiting examples of consumer product cleaning compositions include light duty liquid deterents (LDL), heavy duty liquid detergents (HDL), heavy duty granular detergents (HDG), softergents (STW), hard surface cleaners (HSC), bar soaps, fabric softeners (FS), and special purpose cleaners (SPC). Any of the aforementioned examples of consumer product cleaning compositions can optionally include perfume, as described in U.S. Patent No. 5,500,154 and WO 96/02490.

Light duty liquid detergents include compositions having surfactancy improving magnesium ions (see, e.g., WO 97/00930A; GB 2,292,562A; U.S. Patent Nos. 5,376,310; 5,269,974; 5,230,823; 4,923,635; 4,681,704; 4,316,824; 4,133,779), organic diamines, various foam stabilizers, foam boosters such as amine oxides (see, e.g., U.S. Patent No. 4,133,779), skin feel modifiers of surfactant, emollient, and enzymatic types including proteases, antimicrobial agents, and mixtures thereof (see, e.g., Surfactant Science Series, Vol. 67, pages 240-248). Heavy duty liquid detergents include both "structured" (i.e., multi-phase) liquid types (see, e.g., U.S. Patent Nos. 4,452,717; 4,526,709; 4,530,780; 4,618,446; 4,793,943; 4,659,497; 4,871,467; 4,891,147; 5,006,273; 5,021,195; 5,147,576; 5,160,655) and "non-structured" (i.e., isotropic) liquid types, and can be aqueous or nonaqueous (see, e.g., EP 738,778A; WO 97/00937A; WO 97/00936A; EP 752,466A; DE 19623623A; WO 96/10073A; WO 96/10072A; EP 225,654; WO 94/23009; U.S. Patent Nos. 4,647,393; 4,648,983; 4,655,954; 4,661,280; 4,690,771; 4,744,916; 4,753,750; 4,950,424; 5,004,556; and 5,102,574). The HDLs can optionally comprise bleach (see, e.g., U.S. Patent Nos. 4,470,919; 5,250,212; 5,264,143; 5,275,753; 5,288,746; 5,431,848; and 5,445,756; EP 564,250; WO 94/11483; EP 598,170; EP 598,973; and EP 619,368). Additionally or alternatively, the HDLs can optionally comprise enzymes (see, e.g., U.S. Patent Nos. 3,944,470; 4,111,855; 4,261,868; 4,287,082; 4,305,837; 4,404,115; 4,462,922; 4,529,5225; 4,537,706; 4,537,707; 4,670,179; 4,842,758; 4,900,475; 4,908,150; 5,082,585; 5,156,773; 5,269,960; 5,422,030; 5,431,842; and 5,442,100; WO 92/19709; EP 583,534; EP 583,535; EP 583,536; WO 94/04542; and EP 633,311). Also see Surfactant Science Series, Vol. 67, pages 309-324. Heavy duty granular detergents include both the "compact" (i.e., agglomerated or otherwise non-spray-dried) type, and the "fluffy" (i.e., spray-dried) type. The compact and fluffy types of HDGs either can be phosphated or nonphosphated. The HDGs can include the anionic-surfactant based type or the "high-nonionic surfactant" type (i.e., the nonionic surfactant is held in or on an absorbent, such as zeolites or other porous inorganic salts). Manufacture of HDGs is disclosed in, e.g., EP 753,571A; WO 96/38531A; U.S. Patent Nos. 5,576,285; 5,573,697; 5,569,645; 5,565,422; 5,496,487; 5,489,392; and 5,554,587; U.S. Patent Application NO. 96/34082A; EP 739,977A; EP 737,739A; WO 96/27655A; WO 96/25482A; WO 96/23048A; WO 96/22352A; EP 709,449A; WO 96/09370A; and EP 694,608A. Softergents include various granular or liquid softening-through-the wash types of product and can include organic (e.g., quaternary) or inorganic (e.g., clay) softeners (see, e.g., U.S. Patent Nos. 4,140,641; 4,639,321; 4,751,008; 4,844,821; 4,844,824; 4,873,001; 4,911,852; and 5,017,296; EP 753,569A; EP 315,126; and EP 422,787). Hard surface cleaners include all-purpose cleaners, such as, for example, cream cleansers, liquid cleaners, and spray cleaners (e.g., glass cleaners, tile cleaners, bleach spray cleaners); and bathroom cleaners (e.g., mildew-removing, bleach-containing, antimicrobial, acidic type, neutral type, basic types). See, for example, EP 743,280A; EP 743,279A, and WO 96/34938 A. Bar soaps include laundry bars. The bar soaps encompass both the synthetic detergent (i.e., syndet) type, the soap-based type, and types with softener (see, e.g., WO 96/35772A; U.S. Patent No. 5,500,137; and WO96/01889A). These compositions can include those made by common soap-making techniques, such as plodding, and/or more unconventional techniques, such as casting, absorption of surfactant into a porous support, or the like. Other bar soaps, such as those described in BR 9502668; WO 96/04361A; WO 96/04360A; and U.S. Patent No. 5,540,852 are also included, as well as other handwash detergents, such as those described in GB 2,292,155 A and WO 96/01306 A. Fabric softeners include both the conventional liquid and liquid concentrate types (see, e.g., EP 754,749A; WO 96/21715A; EP 705,900A; U.S. Patent Nos. 5,531,910 and 5,500,138), as well as dryer-added or substrate-supported types (see, e.g., U.S. Patent Nos. 5,562,847 and 5,559,088; and EP 704,522A, each incorporated herein by reference). Other fabric softeners include solids, as described in, for example, U.S. Patent No. 5,505,866. Special purpose cleaners include home dry cleaning systems (see, e.g., WO 96/30583A; WO 96/30472A; WO 96/30471A; U.S. Patent No. 5,547,476; WO 96/37652 A); bleach pretreatment products for laundry (see, e.g., EP 751,210 A); fabric care pretreatment products (see, e.g., EP 752,469 A); liquid fine fabric detergent types, especially the high-foaming variety; rinse-aids for dishwashing; liquid bleaches including both chlorine type and oxygen bleach type; disinfecting agents; car or carpet cleaners or shampoos (see, e.g., EP 751,213A; WO 96/15308A); metal cleaners; cleaning auxiliaries (e.g., bleach additives, stain-sticks, pre-treatments including special foam type cleaners, as described in EP 753,560A; EP 753,559A; EP 753,558A; EP 753,557A; EP 753,556A); and anti-sunfade treatments (see, e.g., WO 96/03486A; WO 96/03481A; WO 96/03369A). Consumer product cleaning compositions, can be formulated into a wide range of forms including, for example, powders, liquids, granules, gels, pastes, tablets, pouches, bars, types delivered in dual-compartment containers, spray or foam detergents and other homogeneous or multiphasic consumer cleaning product forms.

The consumer product compositions can be applied by hand in unitary or freely alterable dosage, or by automatic dispensing means. The consumer product compositions are useful in appliances, (e.g., washing machines, dishwashers), in institutional cleaning contexts (e.g., personal cleansing in public facilities), for bottle washing, for surgical instrument cleaning, and/or for cleaning electronic components. The consumer product compositions can have a wide pH range (e.g., about 2 to about 12, or higher), and a wide range of alkalinity reserve. For example, the consumer product compositions can be used in very high alkalinity reserves, such as drain unblocking, in which tens of grams of NaOH equivalent can be present per 100 grams of formulation. These mixtures can also be used in medium alkalinity reserves having 1 to 10 grams of NaOH equivalent, and mild or low-alkalinity ranges (e.g, liquid hand cleaners; acidic, hard-surface cleaners). Both high-foaming and low-foaming detergent types are encompassed.

### Cleaning Components

A cleaning component is a material required to transform a composition containing only the minimum essential ingredients into a composition useful for laundry or cleaning purposes. The cleaning components are easily recognizable to those of skill in the art as being characteristic of laundry or cleaning products. The precise nature of these cleaning components, and levels of incorporation thereof, depends on the physical form of the composition and the nature of the cleaning operation for which it is to be used.

If the cleaning component is used with bleach, it should have good stability. In some embodiments, the cleaning compositions should be boron-free and/or phosphate-free, as required by legislation. The cleaning component(s) can be present in the cleaning composition in an amount of about 0.001 wt.% to about 99.999 wt.%, typically about 70 wt.% to about 95 wt.%, based on the total weight of the cleaning composition. When used for a particular application, the concentration of the cleaning composition can vary widely ranging, for example, from a few parts per million solution to direct application of the neat cleaning composition.

Common cleaning components include, for example, a builder, a surfactant, an enzyme, an enzyme stabilizing system, a polymer, bleach, a bleach activator, a catalytic material, a polymeric soil release agent, a clay soil removal/anti-redeposition agent, a polymeric dispersing agent, a brightener, a dyes or a fabric hueing agent, a dye transfer inhibiting agent, a chelating agent, a thickener, a fabric softener, a perfume, an active ingredient, a carrier, a hydrotrope, a processing aid, a dye or a pigment, a solvent for liquid formulations, a solid filler for bar compositions, color speckles, silvercare, an anti-tarnish and/or anti-corrosion agent, a germicide, an alkalinity source, an anti-oxidant, a pro-perfumes, a solubilizing agent, and mixtures thereof.

In some embodiments, the cleaning compositions (e.g., laundry detergents, laundry detergent additives, hard surface cleaners, synthetic and soap-based laundry bars, fabric softeners and fabric treatment liquids, solids and treatment articles of all kinds) include several cleaning components. In some embodiments, the cleaning compositions include only one or two cleaning components, such as a bleach additive and a surfactant. A comprehensive list of suitable cleaning components and methods is described in U.S. Patent No. 6,593,285.

### Builders

Detergent builders selected from aluminosilicates and silicates are can be included in the compositions herein, for example to assist in controlling mineral, especially calcium and/or magnesium hardness in wash water, or to assist in the removal of particulate soils from surfaces. Also suitable for use herein are synthesized crystalline ion exchange materials or hydrates thereof, an anhydride form: x(M₂O)·ySiO₂·zM'O wherein M is Na and/or K, M' is Ca and/or Mg; y/x is 0.5 to 2.0 and z/x is 0.005 to 1.0 as taught in U.S. Patent No. 5,427,711. Detergent builders in place of or in addition to the silicates and aluminosilicates described hereinbefore can optionally be included in the compositions herein, for example to assist in controlling mineral, especially calcium and/or magnesium hardness in wash water or to assist in the removal of particulate soils from surfaces.

Builder level can vary widely depending upon end use and physical form of the composition. Built detergents typically comprise at least about 1 wt.% builder, based on the total weight of the detergent. Liquid formulations typically comprise about 5 wt.% to about 50 wt.%, more typically 5 wt.% to 35 wt.% of builder to the total weight of the detergent. Granular formulations typically comprise from about 10% to about 80%, more typically 15% to 50% builder by weight of the detergent composition. Lower or higher levels of builders are not excluded. For example, certain detergent additive or high-surfactant formulations can be unbuilt.

Suitable builders herein can be selected from the group consisting of phosphates and polyphosphates, especially the sodium salts; carbonates, bicarbonates, sesquicarbonates and carbonate minerals other than sodium carbonate or sesquicarbonate; organic mono-, di-, tri-, and tetracarboxylates especially water-soluble nonsurfactant carboxylates in acid, sodium, potassium or alkanolammonium salt form, as well as oligomeric or water-soluble low molecular weight polymer carboxylates including aliphatic and aromatic types; and phytic acid. These may be complemented by borates, e.g., for pH-buffering purposes, or by sulfates, especially sodium sulfate and any other fillers or carriers which may be important to the engineering of stable surfactant and/or builder-containing detergent compositions.

### Detersive Surfactants

In some embodiments, the cleaning compositions can further comprise additional surfactants, herein also referred to as co-surfactants. The cleaning composition typically comprise about 0.1% to about 55%, preferably from about 0.5% to about 15%, by weight of co-surfactants. (e.g., anionic co-surfactants, nonionic co-surfactants, cationic co-surfactants). It is to be understood that the mixtures of bio-based C₁₀-C₁₄ linear alkylphenyl sulfonate prepared in the manner of the present invention may be used singly in cleaning compositions or in combination with other detersive surfactants. Typically, fully-formulated cleaning compositions will contain a mixture of surfactant types in order to obtain broad-scale cleaning performance over a variety of soils and stains, and under a variety of usage conditions. One advantage of the C₁₀-C₁₄ linear alkylphenyl sulfonates herein is their ability to be readily formulated in combination with other known surfactant types. Nonlimiting examples of additional surfactants which may be used herein typically at levels from about 1% to about 55%, by weight, include the unsaturated sulfates, the C₁₀-C₁₈ alkyl alkoxy, C₁₀-C₁₈ alkyl alkoxy carboxylates, the C₁₀-C₁₈ glycerol ether sulfates, the C₁₀-C₁₈ alkyl polyglycosides and their corresponding sulfated polyglycosides, and C₁₂-C₁₈ alpha-sulfonated fatty acid esters. Nonionic surfactants such as the ethoxylated C₁₀-C₁₈ alcohols and alkyl phenols can also be used. If desired, other conventional surfactants such as the C₁₂-C₁₈ betaines and sulfobetaines ("sultaines"), C₁₀-C₁₈ amine oxides, and the like, can also be included in the overall compositions. The C₁₀-C₁₈ N-alkyl polyhydroxy fatty acid amides can also be used. See WO 9,206,154. Other sugar-derived surfactants include the N-alkoxy polyhydroxy fatty acid amides. The N-propyl through N-hexyl C₁₂-C₁₈ glucamides can be used for low sudsing. C₁₀-C₂₀ conventional soaps may also be used. If high sudsing is desired, the branched-chain C₁₀-C₁₆ soaps may be used.

A wide range of these co-surfactants can be used in the detergent compositions. A typical listing of anionic, nonionic, ampholytic and zwitterionic classes, and species of these co-surfactants, is given in U.S. Patent No. 3,664,961. Amphoteric surfactants are also described in detail in "Amphoteric Surfactants, Second Edition", E. G. Lomax, Editor (published 1996, by Marcel Dekker, Inc.).

### Amine-Neutralized Anionic Surfactants

Anionic surfactants and adjunct anionic cosurfactants may be neutralized by amines or, preferably, alkanolamines, and alkanolamines are preferred. Suitable non-limiting examples including monoethanolamine, triethanolamine, and other alkanolamines known in the art.

### Enzymes

Enzymes can be included in the present cleaning compositions for a variety of purposes, including removal of protein-based, carbohydrate-based, or triglyceride-based stains from substrates, for the prevention of refugee dye transfer in fabric laundering, and for fabric restoration. Suitable enzymes include proteases, amylases, lipases, cellulases, peroxidases, and mixtures thereof of any suitable origin, such as vegetable, animal, bacterial, fungal and yeast origin. Preferred selections are influenced by factors such as pH-activity and/or stability optima, thermostability, and stability to active detergents, builders and the like. In this respect bacterial or fungal enzymes are preferred, such as bacterial amylases and proteases, and fungal cellulases. Enzymes are normally incorporated into cleaning compositions at levels sufficient to provide a "cleaning-effective amount." The term "cleaning effective amount" refers to any amount capable of producing a cleaning, stain removal, soil removal, whitening, deodorizing, or freshness improving effect on substrates such as fabrics, dishware and the like. In practical terms for current commercial preparations, typical amounts are up to about 5 mg by weight, more typically 0.01 mg to 3 mg, of active enzyme per gram of the consumer product cleaning composition. Stated otherwise, the compositions herein will typically comprise from 0.001% to 5%, preferably 0.01 %-1 % by weight of a commercial enzyme preparation.

A range of enzyme materials and means for their incorporation into synthetic detergent compositions is disclosed in WO 9307263 A; WO 9307260 A; WO 8908694 A; U.S. Patent Nos. 3,553,139; 4,101,457; and 4,507,219. Enzyme materials useful for liquid detergent formulations, and their incorporation into such formulations, are disclosed in U.S. Patent No. 4,261,868.

### Enzyme Stabilizing System

Enzymes for use in detergents can be stabilized by various techniques. Enzyme stabilization techniques are disclosed and exemplified in U.S. Patent Nos. 3,600,319 and 3,519,570; EP 199,405, EP 200,586; and WO 9401532 A. Thus, the enzyme-containing compositions herein may optionally also comprise from about 0.001% to about 10%, preferably from about 0.005% to about 8%, most preferably from about 0.01% to about 6%, by weight of an enzyme stabilizing system. The enzyme stabilizing system can be any stabilizing system which is compatible with the detersive enzyme. Such a system may be inherently provided by other formulation actives, or be added separately, e.g., by the formulator or by a manufacturer of detergent-ready enzymes. Such stabilizing systems can, for example, comprise calcium ion, boric acid, propylene glycol, short chain carboxylic acids, boronic acids, and mixtures thereof, and are designed to address different stabilization problems depending on the type and physical form of the cleaning composition.

### Bleaching Compounds, Bleaching Agents, Bleach Activators, and Bleach Catalysts

In some embodiments, the cleaning compositions can further contain bleaching agents or bleaching compositions containing a bleaching agent and one or more bleach activators. Bleaching agents will typically be present at levels of about 1 wt.% to about 30 wt.%, more typically from about 5 wt.% to about 20 wt.%, based on the total weight of the composition, especially for fabric laundering. If present, the amount of bleach activators will typically be about 0.1 wt.% to about 60 wt.%, more typically about 0.5 wt.% to about 40 wt.% of the bleaching composition comprising the bleaching agent-plus-bleach activator.

Examples of bleaching agents include oxygen bleach, perborate bleach, percarboxylic acid bleach and salts thereof, peroxygen bleach, persulfate bleach, percarbonate bleach, and mixtures thereof. Examples of bleaching agents are disclosed in U.S. Patent No. 4,483,781, U.S. patent application Ser. No. 740,446, European Patent Application 0,133,354, U.S. Patent No. 4,412,934, and U.S. Patent No. 4,634,551. Examples of bleach activators (e.g., acyl lactam activators) are disclosed in U.S. Patent Nos. 4,915,854; 4,412,934; 4,634,551; 4,634,551; and 4,966,723.

In some embodiments, a laundry detergent composition comprises a transition metal catalyst. Preferably, the transition metal catalyst may be encapsulated. The transition metal bleach catalyst typically comprises a transition metal ion, preferably selected from transition metal selected from the group consisting of Mn(II), Mn(III), Mn(IV), Mn(V), Fe(II), Fe(III), Fe(IV), Co(I), Co(II), Co(III), Ni(I), Ni(II), Ni(III), Cu(I), Cu(II), Cu(III), Cr(II), Cr(III), Cr(IV), Cr(V), Cr(VI), V(III), V(IV), V(V), Mo(IV), Mo(V), Mo(VI), W(IV), W(V), W(VI), Pd(II), Ru(II), Ru(III), and Ru(IV), more preferably Mn(II), Mn(III), Mn(IV), Fe(II), Fe(III), Cr(II), Cr(III), Cr(IV), Cr(V), and Cr(VI). The transition metal bleach catalyst typically comprises a ligand, preferably a macropolycyclic ligand, more preferably a cross-bridged macropolycyclic ligand. The transition metal ion is preferably coordinated with the ligand. Preferably, the ligand comprises at least four donor atoms, at least two of which are bridgehead donor atoms. Suitable transition metal bleach catalysts are described in U.S. 5,580,485, U.S. 4,430,243; U.S. 4,728,455; U.S. 5,246,621; U.S. 5,244,594; U.S. 5,284,944; U.S. 5,194,416; U.S. 5,246,612; U.S. 5,256,779; U.S. 5,280,117; U.S. 5,274,147; U.S. 5,153,161; U.S. 5,227,084; U.S. 5,114,606; U.S. 5,114,611, EP 549,271 A1; EP 544,490 A1; EP 549,272 A1; and EP 544,440 A2. A suitable transition metal bleach catalyst is a manganese-based catalyst, for example disclosed in U.S. 5,576282. Suitable cobalt bleach catalysts are described, for example, in U.S. 5,597,936 and U.S. 5,595,967. Such cobalt catalysts are readily prepared by known procedures, such as taught for example in U.S. 5,597,936, and U.S. 5,595,967. A suitable transition metal bleach catalyst is a transition metal complex of ligand such as bispidones described in WO 05/042532 A1. Bleaching agents other than oxygen bleaching agents are also known in the art and can be utilized herein (e.g., photoactivated bleaching agents such as the sulfonated zinc and/or aluminum phthalocyanines (U.S. Patent No. 4,033,718), or preformed organic peracids, such as peroxycarboxylic acid or salt thereof, or a peroxysulphonic acid or salt thereof. A suitable organic peracid is phthaloylimidoperoxycaproic acid. If used, consumer product cleaning compositions will typically contain from about 0.025% to about 1.25%, by weight, of such bleaches, especially sulfonate zinc phthalocyanine.

### Polymeric Soil Release Agent

Known polymeric soil release agents, hereinafter "SRA" or "SRA's", can optionally be employed in the present cleaning compositions. If utilized, SRA's will generally comprise about 0.01% to about 10.0%, typically about 0.1% to about 5%, preferably about 0.2% to about 3.0% by weight, of the composition.

Preferred SRA's typically have hydrophilic segments to hydrophilize the surface of hydrophobic fibers, such as polyester and nylon, and hydrophobic segments to deposit upon hydrophobic fibers and remain adhered thereto through completion of washing and rinsing cycles, thereby serving as an anchor for the hydrophilic segments. This can enable stains occurring subsequent to treatment with SRA to be more easily cleaned in later washing procedures.

SRA's can include, for example, a variety of charged, e.g., anionic or even cationic (see U.S. Patent No. 4,956,447), as well as noncharged monomer units, and structures may be linear, branched or even star-shaped. They may include capping moieties which are especially effective in controlling molecular weight or altering the physical or surface-active properties. Structures and charge distributions may be tailored for application to different fiber or textile types and for varied detergent or detergent additive products. Examples of SRAs are described in U.S. Patent Nos. 4,968,451; 4,711,730; 4,721,580; 4,702,857; 4,877,896; 3,959,230; 3,893,929; 4,000,093; 5,415,807; 4,201,824; 4,240,918; 4,525,524; 4,201,824; 4,579,681; and 4,787,989; European Patent Application 0 219 048; 279,134 A; 457,205 A; and DE 2,335,044.

### Clay Soil Removal/Anti-Redeposition Agents

The compositions can also optionally contain water-soluble ethoxylated amines having clay soil removal and antiredeposition properties. Granular detergent compositions which contain these compounds typically contain about 0.01% to about 10.0%, by weight, of the water-soluble ethoxylates amines; liquid detergent compositions typically contain about 0.01% to about 5% by weight of these compounds.

Exemplary clay soil removal and antiredeposition agents are described in U.S. Patent Nos. 4,597,898; 548,744; 4,891,160; European Patent Application Nos. 111,965; 111,984; 112,592; and WO 95/32272.

### Polymeric Dispersing Agents

Polymeric dispersing agents can advantageously be utilized at levels of about 0.1% to about 7%, by weight, in the compositions herein, especially in the presence of zeolite and/or layered silicate builders. Suitable polymeric dispersing agents include polymeric polycarboxylates and polyethylene glycols, although others known in the art can also be used. It is believed, though it is not intended to be limited by theory, that polymeric dispersing agents enhance overall detergent builder performance, when used in combination with other builders (including lower molecular weight polycarboxylates) by crystal growth inhibition, particulate soil release peptization, and anti-redeposition. Examples of polymeric dispersing agents are found in U.S.

Patent No. 3,308,067, European Patent Application No. 66915, EP 193,360, and EP 193,360.

### Alkoxylated Polyamines

Soil suspension, grease cleaning, and particulate cleaning polymers may include the alkoxylated polyamines. Such materials include but are not limited to ethoxylated polyethyleneimine, ethoxylated hexamethylene diamine, and sulfated versions thereof. A useful example is 600g/mol polyethyleneimine core ethoxylated to 20 EO groups per NH and is available from BASF.

### Brightener

Any optical brighteners or other brightening or whitening agents known in the art can be incorporated at levels typically of about 0.01 % to about 1.2%, by weight, into the cleaning compositions herein. Commercial optical brighteners which may be useful can be classified into subgroups, which include, but are not necessarily limited to, derivatives of stilbene, pyrazoline, coumarin, carboxylic acid, methinecyanines, dibenzothiophene-5,5-dioxide, azoles, 5- and 6-membered-ring heterocycles, and other miscellaneous agents. Examples of such brighteners are disclosed in "The Production and Application of Fluorescent Brightening Agents", M. Zahradnik, Published by John Wiley & Sons, New York (1982). Specific examples of optical brighteners which are useful in the present compositions are those identified in U.S. Patent No. 4,790,856 and U.S. Patent No. 3,646,015.

### Fabric Hueing Agents

The compositions may include fabric hueing agents. Non-limiting examples include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof. In another aspect, suitable polymeric dyes include polymeric dyes selected from the group consisting of fabric-substantive colorants sold under the name of LIQUITINT® (Milliken, Spartanburg, South Carolina, USA), dye-polymer conjugates formed from at least one reactive dye and a polymer selected from the group consisting of polymers comprising a moiety selected from the group consisting of a hydroxyl moiety, a primary amine moiety, a secondary amine moiety, a thiol moiety and mixtures thereof. In still another aspect, suitable polymeric dyes include polymeric dyes selected from the group consisting of LIQUITINT t® (Milliken, Spartanburg, South Carolina, USA) Violet CT, carboxymethyl cellulose (CMC) conjugated with a reactive blue, reactive violet or reactive red dye such as CMC conjugated with C.I. Reactive Blue 19, sold by Megazyme, Wicklow, Ireland under the product name AZO-CM-CELLULOSE, product code S-ACMC, alkoxylated triphenylmethane polymeric colourants, alkoxylated thiophene polymeric colourants, and mixtures thereof.

### Dye Transfer Inhibiting Agents

The compositions may also include one or more materials effective for inhibiting the transfer of dyes from one fabric to another during the cleaning process. Generally, such dye transfer inhibiting agents include polyvinyl pyrrolidone polymers, polyamine N-oxide polymers, copolymers of N-vinylpyrrolidone and N-vinylimidazole, manganese phthalocyanine, peroxidases, and mixtures thereof. If used, these agents typically comprise about 0.01% to about 10% by weight of the composition, preferably from about 0.01% to about 5%, and more preferably from about 0.05% to about 2%.

### Chelating Agents

The detergent compositions herein may also optionally contain one or more iron and/or manganese chelating agents. Such chelating agents can be selected from the group consisting of amino carboxylates, amino phosphonates, polyfunctionally-substituted aromatic chelating agents and mixtures therein. If utilized, these chelating agents will generally comprise about 0.1 % to about 15% by weight of the detergent compositions herein. More preferably, if utilized, the chelating agents will comprise from 0.1% to about 3.0% by weight of such compositions.

### Stucturant / Thickeners

Structured liquids can either be internally structured, whereby the structure is formed by primary ingredients (e.g. surfactant material) and/or externally structured by providing a three dimensional matrix structure using secondary ingredients (e.g. polymers, clay and/or silicate material). The composition may comprise a structurant in an amount of about 0.01 wt.% to 5 wt.%, preferably about 0.1 wt.% to 2.0 wt.%, based on the total weight of the composition. The structurant is typically selected from the group consisting of diglycerides and triglycerides, ethylene glycol distearate, microcrystalline cellulose, cellulose-based materials, microfiber cellulose, biopolymers, xanthan gum, gellan gum, and mixtures thereof. A suitable structurant includes hydrogenated castor oil, and non-ethoxylated derivatives thereof. A suitable structurant is disclosed in US Patent No. 6,855,680. Such structurants have a thread-like structuring system having a range of aspect ratios. Other suitable structurants and the processes for making them are described in WO2010/034736.

### Alkoxylated Polycarboxylates

Alkoxylated polycarboxylates, such as those prepared from polyacrylates, are useful herein to provide additional grease removal performance. Such materials are described in WO 91/08281 and PCT 90/01815. Chemically, these materials comprise polyacrylates having one ethoxy side-chain per every 7-8 acrylate units. The side-chains are of the formula -(CH2CH2O)m (CH2)nCH3 wherein m is 2-3 and n is 6-12. The side-chains are ester-linked to the polyacrylate "backbone" to provide a "comb" polymer type structure. The molecular weight can vary, but is typically in the range of about 2000 to about 50,000. Such alkoxylated polycarboxylates can comprise about 0.05% to about 10%, by weight, of the compositions herein.

### Amphiphilic graft co-polymer

The mixtures of bio-based C₁₀-C₁₄ linear alkylphenylsulfonates made according to the present invention, and their mixtures with other cosurfactants and other adjunct ingredients, can be used with an amphilic graft co-polymer, preferably the amphilic graft co-polymer comprises (i) polyethyelene glycol backbone; and (ii) and at least one pendant moiety selected from polyvinyl acetate, polyvinyl alcohol and mixtures thereof. A preferred amphilic graft co-polymer is Sokalan HP22, supplied from BASF.

### Fabric Softeners

Various through-the-wash fabric softeners, especially the impalpable smectite clays of U.S. Patent No. 4,062,647, as well as other softener clays known in the art, can optionally be used typically at levels of about 0.5% to about 10%, by weight in the present compositions to provide fabric softener benefits concurrently with fabric cleaning. Clay softeners can be used in combination with amine and cationic softeners as disclosed, for example, in U.S. Patent No. 4,375,416, and U.S. Patent No. 4,291,071.

### Perfumes

Perfumes and perfumery ingredients useful in the present compositions and processes comprise a wide variety of natural and synthetic chemical ingredients, including, but not limited to, aldehydes, ketones, esters, and the like. Also included are various natural extracts and essences which can comprise complex mixtures of ingredients, such as orange oil, lemon oil, rose extract, lavender, musk, patchouli, balsamic essence, sandalwood oil, pine oil, cedar, and the like. Finished perfumes can comprise extremely complex mixtures of such ingredients. Finished perfumes typically comprise about 0.01% to about 2%, by weight, of the detergent compositions herein, and individual lay softeners can be used in combination with amine and cationic softeners perfumery ingredients can comprise about 0.0001% to about 90%, by weight, of a finished perfume composition.

### Other Ingredients

A wide variety of other ingredients useful in the cleaning compositions can be included in the compositions herein, including other active ingredients, carriers, hydrotropes, processing aids, dyes or pigments, solvents for liquid formulations, solid fillers for bar compositions, etc. If high sudsing is desired, suds boosters such as C₁₀-C₁₆ alkanolamides can be incorporated into the compositions, typically at 1%-10% levels. The C₁₀-C₁₃ monoethanol and diethanol amides illustrate a typical class of such suds boosters. Use of such suds boosters with high sudsing adjunct surfactants such as the amine oxides, betaines and sultaines noted above is also advantageous. If desired, water-soluble magnesium and/or calcium salts such as MgCl₂, MgSO₄, CaCl₂, CaSO₄ and the like, can be added at levels of, typically, 0.1%-2%, to provide additional suds and to enhance grease removal performance.

Various detersive ingredients employed in the present compositions optionally can be further stabilized by absorbing said ingredients onto a porous hydrophobic substrate, then coating said substrate with a hydrophobic coating. Preferably, the detersive ingredient is admixed with a surfactant before being absorbed into the porous substrate. In use, the detersive ingredient is released from the substrate into the aqueous washing liquor, where it performs its intended detersive function.

Liquid detergent compositions can contain water and other solvents as carriers. Low molecular weight primary or secondary alcohols exemplified by methanol, ethanol, propanol, and isopropanol are suitable. Monohydric alcohols are preferred for solubilizing surfactant, but polyols such as those containing from 2 to about 6 carbon atoms and from 2 to about 6 hydroxy groups (e.g., 1,3-propanediol, ethylene glycol, glycerine, and 1,2-propanediol) can also be used. The compositions may contain from 5% to 90%, typically 10% to 50% by weight of such carriers.

The cleaning compositions herein will preferably be formulated such that, during use in aqueous cleaning operations, the wash water will have a pH of between about 6.5 and about 11, preferably between about 7.5 and 10.5. Liquid dishwashing product formulations preferably have a pH between about 6.8 and about 9.0. Laundry products are typically at pH 9-11. Techniques for controlling pH at recommended usage levels include the use of buffers, alkalis, acids, etc., and are well known to those skilled in the art.

### Form of the Compositions

The compositions in accordance with the invention can take a variety of physical forms including granular, tablet, bar and liquid forms. Also included are a sachet, a two-in-one pouch containing both solid and liquid compartments. The compositions can be the so-called concentrated granular detergent compositions adapted to be added to a washing machine by means of a dispensing device placed in the machine drum with the soiled fabric load.

The mean particle size of the components of granular compositions in accordance with the invention should preferably be such that no more that 5% of particles are greater than 1.7 mm in diameter and not more than 5% of particles are less than 0.15 mm in diameter.

The term mean particle size as defined herein is calculated by sieving a sample of the composition into a number of fractions (typically 5 fractions) on a series of Tyler sieves. The weight fractions thereby obtained are plotted against the aperture size of the sieves. The mean particle size is taken to be the aperture size through which 50% by weight of the sample would pass.

The bulk density of granular detergent compositions in accordance with the present invention typically have a bulk density of at least 600 g/liter, more preferably from 650 g/liter to 1200 g/liter. Bulk density is measured by means of a simple funnel and cup device consisting of a conical funnel molded rigidly on a base and provided with a flap valve at its lower extremity to allow the contents of the funnel to be emptied into an axially aligned cylindrical cup disposed below the funnel. The funnel is 130 mm high and has internal diameters of 130 mm and 40 mm at its respective upper and lower extremities. It is mounted so that the lower extremity is 140 mm above the upper surface of the base. The cup has an overall height of 90 mm, an internal height of 87 mm and an internal diameter of 84 mm. Its nominal volume is 500 mm.

To carry out a measurement, the funnel is filled with powder by hand pouring, the flap valve is opened and powder allowed to overfill the cup. The filled cup is removed from the frame and excess powder removed from the cup by passing a straight edged implement e.g.; a knife, across its upper edge. The filled cup is then weighed and the value obtained for the weight of powder doubled to provide a bulk density in g/L. Replicate measurements are made as required.

### Surfactant Agglomerate Particles

One of the preferred methods of delivering surfactant in consumer products is to make surfactant agglomerate particles, which may take the form of flakes, prills, marumes, noodles, ribbons, but preferably take the form of granules. A preferred way to process the particles is by agglomerating powders (e.g. aluminosilicate, carbonate) with high active surfactant pastes and to control the particle size of the resultant agglomerates within specified limits. Such a process involves mixing an effective amount of powder with a high active surfactant paste in one or more agglomerators such as a pan agglomerator, a Z-blade mixer, or more preferably an in-line mixer, such as those manufactured by Schugi (Holland) BV, 29 Chroomstraat 8211 AS, Lelystad, Netherlands, and Gebruder Lödige Maschinenbau GmbH, D-4790 Paderbom 1, Elsenerstrasse 7-9, Postfach 2050, Germany. Most preferably a high shear mixer is used, such as a Lödige CB (Trade Name).

A high active surfactant paste comprising about 50 wt.% to about 95 wt.%, preferably about 70 wt.% to about 85 wt.% of surfactant is typically used. The paste may be pumped into the agglomerator at a temperature high enough to maintain a pumpable viscosity, but low enough to avoid degradation of the anionic surfactants used. A typical operating temperature of the paste includes about 50°C to about 80°C.

### Compacted Liquid or Powder Detergents

The mixtures of the alkylphenyl sulfonates of the invention, and their mixtures with other cosurfactants and other adjunct ingredients, are suited to compact detergent formulations. For liquid detergents, the composition preferably comprises less than about 20 wt.%, or less than about 10 wt.%, or less than about 5 wt.%, or less than about 4 wt.% or less than about 3 wt.% free water, or less than about 2 wt.% free water, or less than about 1 wt.% free water, and may even be anhydrous, typically comprising no deliberately added free water. Free water is typically measured using Karl Fischer titration. The laundry detergent composition (e.g., 2 g) is extracted into 50 mL of dry methanol at room temperature for about 20 minutes and about 1 mL of the solution is analyzed by Karl Fischer titration. For powder detergents, the amount of filler (e.g., sodium sulfate, sodium chloride, clay, or other inert solid ingredients) preferably comprises less than about 20 wt.%, or less than about 10 wt.%, or less than about 5 wt.%, or less than about 4 wt.% or less than about 3 wt.% free water, or less than about 2 wt.% free water, or less than about 1 wt.% filler.

### Laundry Washing Method

In some embodiments, the invention provides a method of laundering soiled fabrics comprising contacting the soiled fabrics with an effective amount of a detergent composition described herein.

Machine laundry methods herein typically comprise treating soiled laundry with an aqueous wash solution in a washing machine having dissolved or dispensed therein an effective amount of a machine laundry detergent composition in accord with the invention. By an effective amount of the detergent composition it is meant from 20 g to 300 g of product dissolved or dispersed in a wash solution of volume from 5 to 65 liters, as are typical product dosages and wash solution volumes commonly employed in conventional machine laundry methods.

As noted, the mixtures of bio-based C₁₀-C₁₄ linear alkylphenyl sulfonates having particular distributions are used herein in cleaning compositions, preferably in combination with other detersive surfactants, at levels which are effective for achieving at least a directional improvement in cleaning performance. In the context of a fabric laundry composition, such "usage levels" can vary depending not only on the type and severity of the soils and stains, but also on the wash water temperature, the volume of wash water and the type of washing machine (e.g., top-loading, front-loading, top-loading, vertical-axis Japanese-type automatic washing machine)

As can be seen from the foregoing, the mixtures of bio-based C₁₀-C₁₄ linear alkylphenyl sulfonates having particular distributions used in a machine-wash laundering context can vary, depending on the habits and practices of the user, the type of washing machine, and the like. In this context, however, one heretofore unappreciated advantage of the mixtures of alkylphenyl sulfonates having particular distributions is their ability to provide at least directional improvements in performance over a spectrum of soils and stains, even when used at relatively low levels with respect to the other surfactants (generally anionics or anionic/nonionic mixtures) in the finished compositions.

In addition, another advantage of the mixtures of bio-based C₁₀-C₁₄ linear alkylphenyl sulfonates having particular distributions and the detergent compositions containing them is their desirable performance in cold water. The invention herein includes methods for laundering of fabrics at reduced wash temperatures. This method of laundering fabric comprises the step of contacting a laundry detergent composition to water to form a wash liquor, and laundering fabric in said wash liquor, wherein the wash liquor has a temperature of above 0°C to 20°C, preferably to 19°C, or to 18°C, or to 17°C, or to 16°C,or to 15°C, or to 14°C, or to 13°C, or to 12°C, or to 11°C, or to 10°C, or to 9°C, or to 8°C, or to 7°C, or to 6°C, or even to 5°C. The fabric may be contacted to the water prior to, or after, or simultaneous with, contacting the laundry detergent composition with water.

A further method of use of the materials involves pretreatment of stains prior to laundering.

### Hand Machine Dishwashing Methods

Any suitable methods for machine washing or cleaning soiled tableware, particularly soiled silverware are envisaged.

A preferred liquid hand dishwashing method involves either the dissolution of the detergent composition into a recepticle containing water, or by the direct application of the liquid hand dishwashing detergent composition onto soiled dishware.

A preferred machine dishwashing method comprises treating soiled articles selected from crockery, glassware, hollowware, silverware and cutlery and mixtures thereof, with an aqueous liquid having dissolved or dispensed therein an effective amount of a machine dishwashing composition in accord with the invention. By an effective amount of the machine dishwashing composition it is meant from 8 g to 60 g of product dissolved or dispersed in a wash solution of volume from 3 to 10 liters, as are typical product dosages and wash solution volumes commonly employed in conventional machine dishwashing methods.

### Cleansing Hard Surfaces

Any suitable methods for cleaning hard surfaces, such as wood, ceramic, glass, marble, porcelain, grout or concrete using the compositions described herein are envisaged. In some embodiments, an effective amount of a detergent composition of the invention is directly applied to the hard surface.

### Packaging for the Compositions

Commercially marketed executions of the bleaching compositions can be packaged in any suitable container including those constructed from paper, cardboard, plastic materials and any suitable laminates. A preferred packaging execution is described in European Application No. 94921505.7.

### Personal Care Compositions

Personal care compositions, which can be aqueous or anhydrous, are described in European

Patent No. 1299080, U.S. Patent Application Publication No. 2009/0232873, and U.S. Patent No. 5,932,202. Non-limiting examples of personal care products include those intended for use with hair or skin such as a shampoo, a hair conditioner, a hair treatment, a facial soap, a body wash, a body soap (liquid or bar), a foam bath, a make-up remover, a skin care product, an acne control product, a deodorant, an antiperspirant, a shaving aid, a cosmetic, a depilatory, a fragrance, special purpose cleaners and mixtures thereof. See, e.g., WO 96/37595A; WO 96/37592A; WO 96/37591A; WO 96/37589A; WO 96/37588A; GB 2,297,975A; GB 2,297,762A; GB 2,297,761A; WO 96/17916A; WO 96/12468A. Personal care cleaning compositions can be formulated into, for example, a wipe, a cloth, a bar, a liquid, a powder, a crème, a lotion, a spray, an aerosol, a foam, a mousse, a serum, a capsule, a gel, an emulsion, a doe foot, a roll-on applicator, a stick, a sponge, an ointment, a paste, an emulsion spray, a tonic, a cosmetic, and mixtures thereof. Products, such as devices, appliances, applicators, implements, combs, brushes, and substrates are also encompassed by the invention. These products can be used alone on the skin or hair, or in combination with the personal care cleaning compositions described herein.

The personal care product of the invention can be applied by hand in unitary or freely alterable dosage, or by automatic dispensing means. The personal care composition of the invention also can be dispensed from an article, such as, for example, a bottle, a jar, a tube, a sachet, a pouch, a container, a tottle, a vial, an ampoule, or a compact, or can be integrally contained within a delivery form, such as a wipe.

In some preferred embodiments, the personal care compositions may be used in direct application to the skin or in a conventional manner for cleansing, treating or conditioning skin and hair. The compositions herein are useful for cleansing or conditioning the hair and scalp, and other areas of the body and for any other area of skin in need of treatment. The present invention may be used for treating, cleansing, or conditioning of the skin or hair of animals as well. An effective amount of the composition, typically from about 1 g to about 50 g, preferably from about 1 g to about 20 g of the composition, for cleansing and/or conditioning hair, skin or other area of the body, is topically applied to the hair, skin or other area that has preferably been wetted, generally with water, and then rinsed off. Application to the hair typically includes working the composition through the hair.

### Personal Care Components

A personal care component is a material required to transform a composition containing only the minimum essential ingredients into a composition useful for personal care purposes. The personal care components are easily recognizable to those of skill in the art as being characteristic of personal care products. The precise nature of these personal care components, and levels of incorporation thereof, depends on the physical form of the composition and the nature of the personal care operation for which it is to be used

The personal component(s) can be present in the personal care composition in an amount of about 0.001 wt.% to about 99.999 wt.%, typically about 70 wt.% to about 95 wt.%, based on the total weight of the personal care composition. When used for a particular application, the concentration of the personal care composition of the invention can vary widely ranging, for example, from a few parts per million solution to direct application of the personal care composition.

Common personal care components include, for example, an oil, an emollient, a moisturizer, a carrier, an extract, a vitamin, a mineral, an anti-aging compound, a surfactant, a solvent, a polymer, a preservative, an antimicrobial, a wax, a particle, a colorant, a dye, a fragrance, and mixtures thereof. In some embodiments, the personal care compositions of the invention (e.g.,) include several personal care components. In some embodiments, the personal care compositions include only one or two personal components, such as a detersive surfactant and a hair conditioning active. Lists of personal care components and methods are described in U.S. Patent Application No.2007/002022 and U.S. Patent No. 5,932,202.

In some embodiments, the personal care composition further includes a detersive surfactant. The detersive surfactant component is included to provide improved cleaning performance to the composition. The detersive surfactant component in turn comprises anionic detersive surfactant, zwitterionic or amphoteric detersive surfactant, or a combination thereof. Such surfactants should be physically and chemically compatible with the essential components described herein, or should not otherwise unduly impair product stability, aesthetics or performance.

Suitable anionic detersive surfactant components for use in the personal care composition herein include those which are known for use in hair care or other personal care cleansing compositions. The concentration of the anionic surfactant component in the composition should be sufficient to provide the desired cleaning and lather performance, and generally range from about 5 wt.% to about 50 wt.%, preferably from about 8 wt.% to about 30 wt.%, more preferably from about 10 wt.% to about 25 wt.%, even more preferably from about 12 wt.% to about 22 wt.%, based on the total weight of the personal care composition..

Preferred anionic surfactants suitable for use in the personal care composition are the alkyl and alkyl ether sulfates. These materials have the respective formulae ROSO₃M and RO(C₂H₄O)ₓSO₃M, wherein R is alkyl or alkenyl of about 8 to about 18 carbon atoms, x is an integer having a value of from 1 to 10, and M is a cation such as ammonium, alkanolamines, such as triethanolamine, monovalent metals, such as sodium and potassium, and polyvalent metal cations, such as magnesium, and calcium.

Preferably, R has about 8 to about 18 carbon atoms, more preferably from about 10 to about 16 carbon atoms, even more preferably from about 12 to about 14 carbon atoms, in both the alkyl and alkyl ether sulfates. The alkyl ether sulfates are typically made as condensation products of ethylene oxide and monohydric alcohols having about 8 to about 24 carbon atoms. The alcohols can be synthetic or they can be derived from fats, e.g., coconut oil, palm kernel oil, tallow. Lauryl alcohol and straight chain alcohols derived from coconut oil or palm kernel oil are preferred. Such alcohols are reacted with about 0 to about 10, preferably about 2 to about 5, more preferably about 3, molar proportions of ethylene oxide, and the resulting mixture of molecular species having, for example, an average of 3 moles of ethylene oxide per mole of alcohol, is sulfated and neutralized.

Other suitable anionic detersive surfactants are the water soluble salts of organic, sulfuric acid reaction products conforming to the formula [R¹SO₃M] where R¹ is a straight or branched chain, saturated, aliphatic hydrocarbon radical having about 8 to about 24, preferably about 10 to about 18, carbon atoms; and M is a cation described hereinbefore.
Still other suitable anionic detersive surfactants are the reaction products of fatty acids esterified with isethionic acid and neutralized with sodium hydroxide where, for example, the fatty acids
are derived from coconut oil or palm kernel oil; sodium or potassium salts of fatty acid amides of methyl tauride in which the fatty acids, for example, are derived from coconut oil or palm kernel oil. Other similar anionic surfactants are described in U.S. Patent Nos. 2,486,921; 2,486,922; and 2,396,278.

Other anionic detersive surfactants suitable for use in the compositions are the succinnates, examples of which include disodium N-octadecylsulfosuccinnate; disodium lauryl sulfosuccinate; diammonium lauryl sulfosuccinate; tetrasodium N-(1,2-dicarboxyethyl)-N-octadecylsulfosuccinnate; diamyl ester of sodium sulfosuccinic acid; dihexyl ester of sodium sulfosuccinic acid; and dioctyl esters of sodium sulfosuccinic acid.

Other suitable anionic detersive surfactants include alkene sulfonates having about 10 to about 24 carbon atoms. In addition to the true alkene sulfonates and a proportion of hydroxy alkanesulfonates, the alkene sulfonates can contain minor amounts of other materials, such as alkene disulfonates depending upon the reaction conditions, proportion of reactants, the nature of the starting alkenes and impurities in the alkene stock and side reactions during the sulfonation process. A nonlimiting example of such an alpha alkene sulfonate mixture is described in U.S. Patent No. 3,332,880.

Another class of anionic detersive surfactants suitable for use in the compositions are the beta-alkyloxy alkane sulfonates. These surfactants conform to the formula: 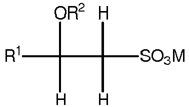 where R¹ is a straight chain alkyl group having about 6 to about 20 carbon atoms, R² is a lower alkyl group having about 1 to about 3 carbon atoms, preferably 1 carbon atom, and M is a water-soluble cation as described hereinbefore.

Preferred anionic detersive surfactants for use in the compositions include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate and combinations thereof.

Suitable amphoteric or zwitterionic detersive surfactants for use in the composition herein include those which are known for use in hair care or other personal care cleansing. Concentration of such amphoteric detersive surfactants preferably are about 0.5 wt.% to about 20 wt.%, preferably about 1 wt.% to about 10 wt.%. Nonlimiting examples of suitable zwitterionic or amphoteric surfactants are described in U.S. Patent Nos. 5,104,646 and 5,106,609.

Amphoteric detersive surfactants suitable for use in the composition are well known in the art, and include those surfactants broadly described as derivatives of aliphatic secondary and tertiary amines in which the aliphatic radical can be straight or branched chain and wherein one of the aliphatic substituents contains about 8 to about 18 carbon atoms and one contains an anionic group such as carboxy, sulfonate, sulfate, phosphate, or phosphonate. Preferred amphoteric detersive surfactants for use in the present invention include cocoamphoacetate, cocoamphodiacetate, lauroamphoacetate, lauroamphodiacetate, and mixtures thereof. Zwitterionic detersive surfactants suitable for use in the composition are well known in the art, and include those surfactants broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group such as carboxy, sulfonate, sulfate, phosphate or phosphonate. Zwitterionics such as betaines are preferred.

The personal care compositions may further comprise additional surfactants for use in combination with the anionic detersive surfactant component described hereinbefore. Suitable optional surfactants include nonionic and cationic surfactants. Any such surfactant known in the art for use in hair or personal care products may be used, provided that the optional additional surfactant is also chemically and physically compatible with the essential components of the composition, or does not otherwise unduly impair product performance, aesthetics or stability. The concentration of the optional additional surfactants in the composition may vary with the cleansing or lather performance desired, the optional surfactant selected, the desired product concentration, the presence of other components in the composition, and other factors well known in the art.

Nonlimiting examples of other anionic, zwitterionic, amphoteric or optional additional surfactants suitable for use in the compositions are described in McCutcheon's, Emulsifiers and Detergents, 1989 Annual, published by M. C. Publishing Co., and U.S. Patent Nos. 3,929,678, 2,658,072; 2,438,091; and 2,528,378.

The personal care compositions can be useful for cleaning and treating a number of mammalian keratinous tissue conditions. Such treatment of keratinous tissue conditions can include prophylactic and therapeutic regulation. More specifically, such treatment methods can be directed to, but are not limited to, preventing, retarding, and/or treating uneven skin tone, reducing the size of pores in mammalian skin, regulating oily/shiny appearance of mammalian skin, thickening keratinous tissue (i.e., building the epidermis and/or dermis and/or subcutaneous layers of the skin and where applicable the keratinous layers of the nail and hair shaft), preventing, retarding, and/or treating uneven skin tone by acting as a lightening or pigmentation reduction cosmetic agent, preventing, retarding, and/or treating atrophy of mammalian skin, softening and/or smoothing lips, hair and nails of a mammal, preventing, retarding, and/or treating itch of mammalian skin, preventing, retarding, and/or treating the appearance of dark under-eye circles and/or puffy eyes, preventing, retarding, and/or treating sallowness of mammalian skin, preventing, retarding, and/or treating sagging (i.e., glycation) of mammalian skin, preventing and/or retarding tanning of mammalian skin, desquamating, exfoliating, and/or increasing turnover in mammalian skin, preventing, retarding, and/or treating hyperpigmentation such as post-inflammatory hyperpigmentation, preventing, retarding, and/or treating the appearance of spider vessels and/or red blotchiness on mammalian skin, preventing, retarding, and/or treating fine lines and wrinkles of mammalian skin, preventing, retarding, and/or treating skin dryness (i.e., roughness, scaling, flaking) and preventing, retarding, and/or treating the appearance of cellulite in mammalian skin. In a preferred embodiment, the personal care composition is used to treat the signs of aging; in one aspect, the composition is used to regulate the signs of aging; in another aspect, the composition is used to reduce or decrease the signs of aging; in yet another aspect the composition is used to prevent the signs of aging in keratinous tissue (e.g., skin, hair, or nails).

For example, the personal care composition can be useful for therapeutically regulating visible and/or tactile discontinuities in mammalian keratinous tissue, including discontinuities in skin texture and color. In some embodiments, the personal care composition can decrease the apparent diameter of pore. In some embodiments, the apparent height of tissue immediately proximate to pore openings can approach that of the interadnexal skin. In other embodiments, the skin tone/color can become more uniform, and/or the length, depth, and/or other dimension of lines and/or wrinkles can be decreased.

Furthermore, the personal care compositions can also be useful for cleansing (e.g., hair, body, facial), improving keratinous tissue feel (wet & dry) such as for hair (e.g., improving appearance/look, detangling, shine, gloss, decrease coefficient of friction, increase smoothness, color retention, decrease split ends, prevent hair breakage, prevent environmental damage such as sunlight damage, smoke damage, and damage from pollutants such as nitrogen oxides, sulfur oxides, ozone, and metals such as lead), odor control, oil control, conditioning, hair volume control, hair growth, and hair growth inhibition.

Regulating keratinous tissue conditions can involve topically applying to the keratinous tissue a safe and effective amount of a personal care composition. The amount of the composition that is applied, the frequency of application, and the period of use will vary widely depending upon the level of components of a given composition and the level of regulation desired, e.g., in view of the level of keratinous tissue damage present or expected to occur.

Furthermore, regulating keratinous tissue conditions can involve orally ingesting a safe and effective amount of a composition. The amount of the composition that is ingested, the frequency of ingestion, and the period of use will vary widely depending upon the level of components of a given composition and the level of regulation desired, e.g., in view of the level of keratinous tissue damage present or expected to occur.

In one embodiment, the personal care composition is chronically applied to the skin, e.g. topically. By "chronic application" is meant continued topical application of the composition over an extended period during the subject's lifetime, preferably for a period of at least about one week, more preferably for a period of at least about one month, even more preferably for at least about three months, even more preferably for at least about six months, and more preferably still for at least about one year. While benefits are obtainable after various maximum periods of use (e.g., five, ten or twenty years), it is preferred that chronic applications continue throughout the subject's lifetime. Typically applications would be on the order of about once per day over such extended periods;, however, application rates can vary, and can include from about once per week up to about three times per day or more.

Treating keratinous tissue condition can be practiced, for example, by applying a composition in the form of a skin lotion, clear lotion, milky lotion, cream, gel, foam, ointment, paste, emulsion, spray, aerosol, conditioner, tonic, cosmetic, lipstick, foundation, nail polish, after-shave, roll-on or deodorant stick, powder, oil or the like which is intended to be left on the skin or rinsed off. Any part of the external portion of the face, hair, and/or nails can be treated, (e.g., face, lips, under-eye area, eyelids, scalp, neck, torso, arms, hands, legs, feet, fingernails, toenails, scalp hair, eyelashes, eyebrows, etc.)

### EXAMPLES

### Example 1a: Synthesis of Individual Chain Length Alkenes Having at Least 50% Bio-Based Carbon Content for Blending to Standard Commercial Distributions Via Alkene Metathesis

The metathesis synthesis procedure of US2010/0145086A1 was followed using one of 2-butene, 3-hexene, 4-octene, or 5-decene and soybean oil to provide C₁₁, C₁₂, C₁₃ or C₁₄ alkenes, respectively, for blending. The C₁₀ alkene needed for blending is obtained via metathesis of 1-hexene and soybean oil; the C₁₄ alkene also formed is easily separated from the C₁₀ by simple distillation and can also be used for blending. The following commercially representative olefin blends are prepared using the individually-produced alkenes as shown in the table below.

| Olefin Chain length | Avg 11.4 | Avg 11.14 | Avg 11.6 | Avg 11.7 | Avg 12.2 |
|---|---|---|---|---|---|
| 10 | 22 | 32 | 10 | 13 | 9.3 |
| 11 | 31.9 | 33.1 | 37.2 | 30.5 | 21.0 |
| 12 | 29.6 | 25.2 | 32.3 | 31.5 | 25.6 |
| 13 | 16.1 | 9.7 | 19.9 | 24.3 | 30.7 |
| 14 | 0.3 | 0 | 0.7 | 0.7 | 13.5 |

### Example 1a1 - Synthesis of An Avg 11.7 Alkene Mixture using Metathesis

By using the right mixture of butenes and hexenes derived from various sources one can prepare a close match to the Avg. 11.7 olefin blend listed in the table above. Thus, reaction of oleic acid with an olefin blend comprising 0.246 molar equivalents of 1-butene, 0.062 molar equivalents of 2-butene, 0.0145 molar equivalents of 1-hexene, 0.486 molar equivalents of 2-hexene, and 0.192 molar equivalents of 3-hexene in the presence of a metathesis catalyst produces an olefin mixture having the chain length distribution noted in the above table for Avg. 11.7.

| Chain length | Molar Equivalents Provided by Each Feed Olefin | Wt. % of Mixture Produced |
|---|---|---|
| | 0.007 from 1-hexene | |
| 10 | 0.246 from 1-butene | 19.6 |
| | 0.243 from 2-hexene | |
| 11 | 0.062 from 2-butene | 26.0 |
| | 0.192 from 3-hexene | |
| 12 | 0.123 from 1-butene | 29.3 |
| 13 | 0.243 from 2-hexene | 24.5 |
| 14 | 0.007 from 1-hexene | 0.8 |

### Example 1b: Synthesis of a Commercial Bio-Based Linear Alkyl Benzene Mixture

The alkene mixtures from Example 1a are alkylated using any of the procedures in known in the art, such as, Alul et al., J. Org. Chem., 32(11):3365-3369 (1967) to prepare the commercial linear alkylbenzenes.

### Example 1c: Synthesis of a Commercial Linear Alkyl Benzene Sulfonic Acid Mixture Containing Bio-Based Carbon

### The commercial mixtures of 1b are sulfonated using the following procedure:

The molecular weight of the bio-based linear alkylbenzene was determined by proton nuclear magnetic resonance. A measured amount of linear alkylbenzene was placed into an NMR tube along with a measured amount of dimethyl carbonate (DMC). The amount of DMC was calculated to provide approximately the same molar quantity of protons as the phenyl protons on the linear alkylbenzene. The molar quantity of the phenyl protons was determined by comparing the integrations of the alkylbenzene and the DMC. The grams of linear alkylbenzene per mole of phenyl groups are determined, followed by the molecular weight, which was 237.6. The linear alkylbenzene should contain less than 1000 ppm of water. If the linear alkylbenzene contains greater than 1000 ppm of water, it should be dried over 4Å molecular sieves over night prior to sulfonation. The sieves can be obtained from any chemical catalog such as Aldrich.

The bio-based linear alkylbenzene (8.5 g; Adjust the amount according to the molecular weight average of the linear alkylbenzene) was placed in a dry, 3-neck, round-bottom flask with magnetic stirring and a thermometer. Anhydrous methylene chloride (about 40 mL) was added to the flask. The mixture is placed into a cooling bath of ice water/NaCl/ethanol and allowed to cool to about -5°C. A dry addition funnel is charged with chlorosulfonic acid (1.03 equivalents relative to the linear alkylbenzene), and the HCl that is generated is scrubbed with a trap containing 1N NaOH. Chlorosulfonic acid is dripped into the flask at a rate that does not allow the temperature of the mix to exceed 10°C. After all chlorosulfonic acid was added, the mixture was stirred at -5°C for about 1 h and then allowed to warm to room temperature. The resulting mixture was transferred to a one-neck round bottom flask and concentrated by rotary evaporation (about 40°C) to remove CH₂Cl₂/HCl.

The resulting mixture was placed in a plastic bottle with about 80 mL of methanol and chilled. The mixture was stirred while about 1.08 equivalents of 25% sodium methoxide was added. The mixture was then concentrated by rotary evaporation to result in a viscous, oily residue. The residue was dissolved in 300 mL of warm water and freeze dried. The final surfactant was collected from the freeze drier. It was a light, tacky material that can be compressed into a gum-like consistency.

### Example 1d: Synthesis of a Commercial Linear Alkyl Benzene Sulfonic Acid Sodium Salt Mixture Containing Bio-Based Carbon

The product of 1c is neutralized with sodium methoxide in methanol and the methanol is evaporated to produce a bio-based linear alkylphenyl sulfonate, sodium salt mixture.

### Example 2a: Synthesis of a Commercially Representative Mixture of Alkenes Having at Least 50% Bio-Based Carbon Content and a alkyl chain average of 11.2 to 12.2

The metathesis synthesis procedure of US2010/0145086A1 was followed using a preblended mixture of short chain olefins (ethylene, 2-butene, 3-hexene, 4-octene, 5-decene) and soybean oil to provide the following olefin blends in one step:

| Olefin Chain length | Avg 11.4 | Avg 11.14 | Avg 11.6 | Avg 11.7 | Avg 12.2 |
|---|---|---|---|---|---|
| 10 | 22 | 32 | 10 | 13 | 9.3 |
| 11 | 31.9 | 33.1 | 37.2 | 30.5 | 21.0 |
| 12 | 29.6 | 25.2 | 32.3 | 31.5 | 25.6 |
| 13 | 16.1 | 9.7 | 19.9 | 24.3 | 30.7 |
| 14 | 0.3 | 0 | 0.7 | 0.7 | 13.5 |

For example, to make the mixture having chain average of about 11.7 mix, 13% ethylene, 30.5% 2-butene, 31.5% 3-hexene, 24.3% 4-octene, 0.7% 5-decene (all by weight) are belnded and metathesized with soybean oil according to the procedure. Incomplete reaction of the 5-decene can result in contamination of the C₁₀ content of the resulting mixture with some non-bio-based C₁₀ olefin. The composition be altered by varying the metathesis reaction conditions and monitoring product mixtures via analysis to produce mixtures listed in the table above.

### Example 2b: Synthesis of a Commercial Bio-Based Linear Alkyl Benzene Mixture

The alkene mixtures from Example 2a are used to alkylate benzene using any of the procedures in known in the art, such as, Alul et al., J. Org. Chem., 32(11):3365-3369 (1967) to prepare the commercial bio-based alkylbenzenes. One could also run the above alkene mixtures through any of the commercially available alkylation units and catalysts.

### Example 2c: Synthesis of a Commercial Linear Alkyl Benzene Sulfonic Acid Mixture Containing Bio-Based Carbon

The commercial mixtures of 2b are sulfonated using the following procedure:
The molecular weight of the bio-based linear alkylbenzene was determined by proton nuclear magnetic resonance. A measured amount of linear alkylbenzene was placed into an NMR tube along with a measured amount of dimethyl carbonate (DMC). The amount of DMC was calculated to provide approximately the same molar quantity of protons as the phenyl protons on the alkylbenzene. The molar quantity of the phenyl protons was determined by comparing the integrations of the linear alkylbenzene and the DMC. The grams of linear alkylbenzene per mole of phenyl groups are determined, followed by the molecular weight, which was 237.6. The linear alkylbenzene should contain less than 1000 ppm of water. If the linear alkylbenzene contains greater than 1000 ppm of water, it should be dried over 4Å molecular sieves over night prior to sulfonation. The sieves can be obtained from any chemical catalog such as Aldrich.

The bio-based linear alkylbenzene (8.5 g; Adjust the amount according to the molecular weight average of the linear alkylbenzene) was placed in a dry, 3-neck, round-bottom flask with magnetic stirring and a thermometer. Anhydrous methylene chloride (about 40 mL) was added to the flask. The mixture is placed into a cooling bath of ice water/NaCl/ethanol and allowed to cool to about -5°C. A dry addition funnel is charged with chlorosulfonic acid (1.03 equivalents relative to the linear alkylbenzene), and the HCl that is generated is scrubbed with a trap containing 1N NaOH. Chlorosulfonic acid is dripped into the flask at a rate that does not allow the temperature of the mix to exceed 10°C. After all chlorosulfonic acid was added, the mixture was stirred at -5°C for about 1 h and then allowed to warm to room temperature. The resulting mixture was transferred to a one-neck round bottom flask and concentrated by rotary evaporation (about 40°C) to remove CH₂Cl₂/HCl.

The resulting mixture was placed in a plastic bottle with about 80 mL of methanol and chilled. The mixture was stirred while about 1.08 equivalents of 25% sodium methoxide was added. The mixture was then concentrated by rotary evaporation to result in a viscous, oily residue. The residue was dissolved in 300 mL of warm water and freeze dried. The final surfactant was collected from the freeze drier. It was a light, tacky material that can be compressed into a gum-like consistency.

### Example 2d: Synthesis of a Commercial Linear Alkyl Benzene Sulfonic Acid Sodium Salt Mixture Containing Bio-Based Carbon

The product of 2c is neutralized with sodium methoxide in methanol and the methanol is evaporated to produce a bio-based linear alkylphenyl sulfonate, sodium salt mixture. This results in a 11.7 average linear alkylsulfonate sodium salt.

### Example 3a - Synthesis of Individual Chain Length Alkenes Having at Least 50% Bio-Based Carbon Content for Blending to Standard Commercial Distributions Via Metabolically Engineered Organisms

*E. Coli* strains were bioengineered according to the modifications described in WO2009/140695A1 to predominantly produce a single C₁₀₋₁₄ alkene/alkane mixture which was isolated from the fermentation tank by extraction and distillation. The following commercially representative blend olefins are then prepared using the individual olefins obtained as shown below.

| Olefin Chain length | Avg 11.4 | Avg 11.14 | Avg 11.6 | Avg 11.7 | Avg 12.2 |
|---|---|---|---|---|---|
| 10 | 22 | 32 | 10 | 13 | 9.3 |
| 11 | 31.9 | 33.1 | 37.2 | 30.5 | 21.0 |
| 12 | 29.6 | 25.2 | 32.3 | 31.5 | 25.6 |
| 13 | 16.1 | 9.7 | 19.9 | 24.3 | 30.7 |
| 14 | 0.3 | 0 | 0.7 | 0.7 | 13.5 |

### Example 3b - Synthesis of a Commercial Bio-Based Linear Alkyl Benzene Mixture

The alkene mixtures from Example 1a are alkylated using any of the procedures in known in the art, such as, Alul et al., J. Org. Chem., 32(11):3365-3369 (1967) to prepare the commercial linear alkylbenzenes.

### Example 3c - Synthesis of a Commercial Linear Alkyl Benzene Sulfonic Acid Mixture Containing Bio-Based Carbon

The commercial mixtures of 1b are sulfonated using the following procedure:
The molecular weight of the bio-based linear alkylbenzene was determined by proton nuclear magnetic resonance. A measured amount of linear alkylbenzene was placed into an NMR tube along with a measured amount of dimethyl carbonate (DMC). The amount of DMC was calculated to provide approximately the same molar quantity of protons as the phenyl protons on the linear alkylbenzene. The molar quantity of the phenyl protons was determined by comparing the integrations of the alkylbenzene and the DMC. The grams of linear alkylbenzene per mole of phenyl groups are determined, followed by the molecular weight, which was 237.6. The linear alkylbenzene should contain less than 1000 ppm of water. If the linear alkylbenzene contains greater than 1000 ppm of water, it should be dried over 4Å molecular sieves over night prior to sulfonation. The sieves can be obtained from any chemical catalog such as Aldrich.

The bio-based linear alkylbenzene (8.5 g; Adjust the amount according to the molecular weight average of the linear alkylbenzene) was placed in a dry, 3-neck, round-bottom flask with magnetic stirring and a thermometer. Anhydrous methylene chloride (about 40 mL) was added to the flask. The mixture is placed into a cooling bath of ice water/NaCl/ethanol and allowed to cool to about -5°C. A dry addition funnel is charged with chlorosulfonic acid (1.03 equivalents relative to the linear alkylbenzene), and the HCl that is generated is scrubbed with a trap containing 1N NaOH. Chlorosulfonic acid is dripped into the flask at a rate that does not allow the temperature of the mix to exceed 10°C. After all chlorosulfonic acid was added, the mixture was stirred at -5°C for about 1 h and then allowed to warm to room temperature. The resulting mixture was transferred to a one-neck round bottom flask and concentrated by rotary evaporation (about 40°C) to remove CH₂Cl₂/HCl.

The resulting mixture was placed in a plastic bottle with about 80 mL of methanol and chilled. The mixture was stirred while about 1.08 equivalents of 25% sodium methoxide was added. The mixture was then concentrated by rotary evaporation to result in a viscous, oily residue. The residue was dissolved in 300 mL of warm water and freeze dried. The final surfactant was collected from the freeze drier. It was a light, tacky material that can be compressed into a gum-like consistency.

### Example 3d - Synthesis of a Commercial Linear Alkyl Benzene Sulfonic Acid Sodium Salt Mixture Containing Bio-Based Carbon

The product of 3c is neutralized with sodium methoxide in methanol and the methanol is evaporated to produce a bio-based linear alkylphenyl sulfonate, sodium salt mixture.

### Example 4a - Synthesis of a Commercially Representative Mixture of Alkenes Having at Least 50% Bio-Based Carbon Content and a alkyl chain average of 11.2 to 12.2

Several *E. coli* strains were bioengineered according to the modifications described in WO2009/140695A1 to provide tailored olefin/paraffin blends per strain. The olefin/paraffins were isolated from the fermentation broths via extraction and distillation to give the following olefin compositions as weight percentages:

| Olefin Chain length | Avg 11.4 | Avg 11.14 | Avg 11.6 | Avg 11.7 | Avg 12.2 |
|---|---|---|---|---|---|
| 10 | 22 | 32 | 10 | 13 | 9.3 |
| 11 | 31.9 | 33.1 | 37.2 | 30.5 | 21.0 |
| 12 | 29.6 | 25.2 | 32.3 | 31.5 | 25.6 |
| 13 | 16.1 | 9.7 | 19.9 | 24.3 | 30.7 |
| 14 | 0.3 | 0 | 0.7 | 0.7 | 13.5 |

### Example 4b - Synthesis of a Commercial Bio-Based Linear Alkyl Benzene Mixture

The alkene mixtures from Example 4a are used to alkylate benzene using any of the procedures in known in the art, such as, Alul et al., J. Org. Chem., 32(11):3365-3369 (1967) to prepare the commercial bio-based alkylbenzenes. One could also run the above alkene mixture through any of the commercially available alkylation units and catalysts.

### Example 4c - Synthesis of a Commercial Linear Alkyl Benzene Sulfonic Acid Mixture Containing Bio-Based Carbon

The commercial mixtures of 4b are sulfonated using the following procedure:
The molecular weight of the bio-based linear alkylbenzene was determined by proton nuclear magnetic resonance. A measured amount of linear alkylbenzene was placed into an NMR tube along with a measured amount of dimethyl carbonate (DMC). The amount of DMC was calculated to provide approximately the same molar quantity of protons as the phenyl protons on the alkylbenzene. The molar quantity of the phenyl protons was determined by comparing the integrations of the linear alkylbenzene and the DMC. The grams of linear alkylbenzene per mole of phenyl groups are determined, followed by the molecular weight, which was 237.6. The linear alkylbenzene should contain less than 1000 ppm of water. If the linear alkylbenzene contains greater than 1000 ppm of water, it should be dried over 4Å molecular sieves over night prior to sulfonation. The sieves can be obtained from any chemical catalog such as Aldrich.

The bio-based linear alkylbenzene (8.5 g; Adjust the amount according to the molecular weight average of the linear alkylbenzene) was placed in a dry, 3-neck, round-bottom flask with magnetic stirring and a thermometer. Anhydrous methylene chloride (about 40 mL) was added to the flask. The mixture is placed into a cooling bath of ice water/NaCl/ethanol and allowed to cool to about -5°C. A dry addition funnel is charged with chlorosulfonic acid (1.03 equivalents relative to the linear alkylbenzene), and the HCl that is generated is scrubbed with a trap containing 1N NaOH. Chlorosulfonic acid is dripped into the flask at a rate that does not allow the temperature of the mix to exceed 10°C. After all chlorosulfonic acid was added, the mixture was stirred at -5°C for about 1 h and then allowed to warm to room temperature. The resulting mixture was transferred to a one-neck round bottom flask and concentrated by rotary evaporation (about 40°C) to remove CH₂Cl₂/HCl.
The resulting mixture was placed in a plastic bottle with about 80 mL of methanol and chilled. The mixture was stirred while about 1.08 equivalents of 25% sodium methoxide was added. The mixture was then concentrated by rotary evaporation to result in a viscous, oily residue. The residue was dissolved in 300 mL of warm water and freeze dried. The final surfactant was collected from the freeze drier. It was a light, tacky material that can be compressed into a gum-like consistency.

### Example 4d - Synthesis of a Commercial Linear Alkyl Benzene Sulfonic Acid Sodium Salt Mixture Containing Bio-Based Carbon

The product of 4c is neutralized with sodium methoxide in methanol and the methanol is evaporated to produce a bio-based linear alkylphenyl sulfonate, sodium salt mixture. This results in a 11.7 average linear alkylsulfonate sodium salt.

### Example 5: Consumer Product Cleaning Formulae Having a Bio-Based Linear Alkylphenyl Sulfonate (LAS) as Primary/Co-Surfactant

The mixture of bio-based C₁₀-C₁₄ linear alkylphenyl sulfonates is added to consumer product cleaning formulations, as shown in the below tables. The LAS can include, for example, a C₁₀-C₁₄ linear alkylphenyl sulfonate having an average chain length of 11.7.

Example 6: Table 1 below shows the results of metathesis of high oleoyl sunflower oil with a mixture of pentenes under standard conditions of room temperature and pressure. Analysis is done by gas chromatography using olefin standards to generate a calibration curve. The percent composition is based on total weight of olefins produced due to both oil self metathesis and cross metathesis of oil with pentenes. Table 1 also shows a standard raw material specification for each chain length based on various commercial, petroleum-derived alkylbenzene mixtures readily available from various suppliers used to make the alkylbenzene sulfonate surfactants used in consumer goods products such as laundry detergents. These raw material specifications are a guide to meeting regulatory requirements for the compositions. Note the importance of choice of short chain olefin co-metathesis feed as well as the ratio of the olefins reacted with the high oleoyl sunflower oil to provide an acceptable composition of the alkene for use in making the alkylbenzene. In some cases as shown below the olefins may require removal of part of the short or long chain fraction of olefin to meet the specification. However, as shown by the last example an on spec mixture is generated without removal of any of the C10 or C 14 fraction by choice of the right blend of olefins and ratio relative to the amount of oil.

**TABLE 1**

| Chain Length | Petroleum Derived Alkyl Benzene | 2-pentene only | 5:1* | 4:1 * |
|---|---|---|---|---|
| | | | 2:1 ** | 2:1** |
| C10 | 16 %*** max | 4.8 % | 17.5 % | 7.5 % |
| C10 + C11 | 30-55 % | 40.6 % | 45.5 % | 34.7% |
| C12 | 20-50 % | 47.7 % | 38.2 % | 42.8 % |
| C13 + C14 | 5-30 % | 11.7 % | 16.4 % | 14.0 % |
| C14 | 4.5 % max | 9.5 % | 7.4 % | 1.1% |

| | | | | |
|---|---|---|---|---|
| *Indicates ratio in moles of olefin feed to moles per moles of unsaturation i.e. olefins in oil feed **Indicates ratio of 2-pentene to 1-pentene ***all percentages are in terms of mass via GC analysis using olefin standards for calibration | | | | |

Example 7: Table 2 below shows the results of experiments using highly unsaturated soybean oil compared to high oleic containing oils. Note the desirable higher percentage of detergent range olefins (DRO's) for the high oleoyl oils and lower undesireable chain length olefins outside of the detergent range for production of acceptable alkylbenzenes. The high oleoyl oil derived olefins are suitable for direct alkylation with benzene without formation of substantial undesireable tetralins and indans and result in high quality alkylbenzenes.

**TABLE 2**

| Information | 2:1* | 2:1 * | 2:1 * |
|---|---|---|---|
| Oil | SBO | High Oleic Sunflower Oil | High Oleic Soybean Oil |
| Hoveyda-Grubbs 2 type catalyst | 800 ppm | 500 ppm | 500 ppm |
| Olefin Feed | pentenes | pentenes | pentenes |
| Olefin Ratio | 5 to 1** | 4 to 1** | 4 to 1** |
| % DROs (C10-C14) | 14.2 | 56.1 | 53.7 |
| % Other Olefins (<C8 not measured) | 50.6 | 16.3 | 14.4 |

| | | | |
|---|---|---|---|
| *Indicates ratio in moles of olefin feed to moles per moles of unsaturation i.e. olefins in oil feed **Indicates ratio of 2-pentene to 1-pentene ***percentages are in terms of mass via GC analysis using olefin standards for calibration | | | |

The following detergent compositions A to K suitable for hand-washing soiled fabrics are prepared in accord with the invention:

**Granular Laundry Detergents**

| | A | B | C | D | E |
|---|---|---|---|---|---|
| Formula | wt% | wt% | wt% | wt% | wt% |
| C₁₀₋₁₄ Linear Alkylbenzene Sulfonic Acid, sodium salt from Example 1d | 5-20 | 5-20 | 5-20 | 5-20 | 5-20 |
| C₁₂₋₁₄ alcohol sulfate, sodium salt | 10-20 | 10-20 | 10-20 | 10-20 | 10-20 |
| C₁₂₋₁₈ Ethoxylate | --- | --- | 0-3 | --- | 0-1 |
| C₁₄₋₁₅ alkyl ethoxylate (EO=7) | 0-3 | 0-3 | --- | 0-5 | 0-3 |
| Dimethyl hydroxyethyl lauryl ammonium chloride | --- | --- | 0-2 | 0-2 | 0-2 |
| Sodium tripolyphosphate | 20 - 40 | --- | 18-33 | 12-22 | 0-15 |
| Zeolite | 0-10 | 20-40 | 0-3 | --- | --- |
| Silicate builder | 0-10 | 0-10 | 0-10 | 0-10 | 0-10 |
| Carbonate | 0-30 | 0-30 | 0-30 | 5-25 | 0-20 |
| Diethylene triamine penta acetate | 0-1 | 0-1 | 0-1 | 0-1 | 0-1 |
| Polyacrylate | 0-3 | 0-3 | 0-3 | 0-3 | 0-3 |
| Carboxy Methyl Cellulose | 0.2-0.8 | 0.2-0.8 | 0.2-0.8 | 0.2-0.8 | 0.2-0.8 |
| Percarbonate | 0-10 | 0-10 | 0-10 | 0-10 | 0-10 |
| Nonanoyloxybenzenesulfonate, sodium salt | --- | --- | 0-2 | 0-2 | 0-2 |
| Tetraacetylethylenediamine | --- | --- | 0-0.6 | 0-0.6 | 0-0.6 |
| Zinc Phthalocyanine Tetrasulfonate | --- | --- | 0-0.005 | 0-0.005 | 0-0.005 |
| Brightener | 0.05-0.2 | 0.05-0.2 | 0.05-0.2 | 0.05-0.2 | 0.05-0.2 |
| MgSO₄ | --- | --- | 0-0.5 | 0-0.5 | 0-0.5 |
| Enzymes | 0-0.5 | 0-0.5 | 0-0.5 | 0-0.5 | 0-0.5 |
| Minors (perfume, dyes, suds stabilizers) | balance | balance | balance | balance | balance |

**Liquid Laundry Detergents**

| Ingredient | F | G | H | I | J | K |
|---|---|---|---|---|---|---|
| | wt.% | wt.% | wt.% | wt.% | wt.% | wt.% |
| C₁₀₋₁₄ Linear Alkylbenzene Sulfonic Acid, sodium salt from Example 1d | 5.5 | 2.7 | 2.2 | 12.2 | 5.2 | 5.2 |
| C₁₂₋₁₄ EO₃ sulfate, sodium salt | 16.5 | 20 | 9.5 | 7.7 | 1.8 | 1.8 |
| Sodium C₁₂₋₁₄ alkyl sulfate, sodium salt | 8.9 | 6.5 | 2.9 | - | | |
| C₁₂₋₁₄ alkyl 7-ethoxylate | | | | | 0.15 | 0.15 |
| C₁₄₋₁₅ alkyl 8-ethoxylate | | | | | 3.5 | 3.5 |
| C₁₂₋₁₅ alkyl 9-ethoxylate | 1.7 | 0.8 | 0.3 | 18.1 | - | - |
| C₁₂₋₁₈ Detergent acid | 2.2 | 2.0 | - | 1.3 | 2.6 | 2.6 |
| Citric acid | 3.5 | 3.8 | 2.2 | 2.4 | 2.5 | 2.5 |
| Protease enzyme | 1.7 | 1.4 | 0.4 | - | 0.5 | 0.5 |
| Amylase enzyme | 0.4 | 0.3 | - | - | 0.1 | 0.1 |
| Mannanase enzyme | | | | | 0.04 | 0.04 |
| PEG-PVAc Polymer¹ | - | - | - | - | - | 0.3 |
| Ethoxyed Hexamethylene Diamine Dimethyl Quat Disulfate | - | - | - | - | - | 0.7 |
| Diethylenetriaminepenta(methylenephosphonic) acid | | | | | 0.2 | 0.2 |
| Solvents (1,2 propanediol, ethanol, stabilizers | 7 | 7.2 | 3.6 | 3.7 | 1.9 | 1.9 |
| Hydrogenated castor oil derivative structurant | 0.3 | 0.2 | 0.2 | 0.2 | 0.35 | 0.35 |
| Polyacrylate | - | - | - | 0.1 | - | - |
| Polyacrylate copolymer² | - | - | - | 0.5 | - | - |
| Sodium carbonate | - | - | - | 0.3 | - | - |
| Sodium silicate | - | - | - | - | - | - |
| Borax | 3 | 3 | 2 | 1.3 | - | - |
| Boric acid | 1.5 | 2 | 2 | 1.5 | 1.5 | 1.5 |
| Perfume | 0.5 | 0.5 | 0.5 | 0.8 | 0.5 | 0.5 |
| Buffers (sodium hydroxide, monoethanolamine) | | | | | 3.3 | 3.3 |
| Water, dyes and miscellaneous | Balance | | | | | |

| | | | | | | |
|---|---|---|---|---|---|---|
| ¹ PEG-PVA graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units. ² Alco 725 (styrene/acrylate) | | | | | | |

### Example 4: Hand Dishwashing Formulae Having a Bio-Based Linear Alkylphenyl Sulfonate (LAS) as Primary/Co-Surfactant

The mixture of bio-based C₁₀-C₁₄ linear alkylphenyl sulfonates is added to hand dishwashing formulations, as shown in the below table. The bio-based C₁₀-C₁₄ linear alkylbenzyl sulfonate having an average chain length of 11.3.

| Formulation/ Component | A | B | C | D |
|---|---|---|---|---|
| LAS from Example 1d | 5 | 10 | 15 | 25 |
| AE(1)S | 15 | 10 | 5 | 5 |
| AS | 10 | 5 | 2 | 0 |
| MES | 0 | 5 | 0 | 0 |
| CMEA | 0.5 | 0 | 0 | 0 |
| CAPB | 1 | 1 | 0 | 1 |
| C11E9 | 0.5 | 2 | 1 | 0 |
| APG | 0 | 0 | 0 | 1.5 |
| Coco Amine oxide | 1 | 0.25 | 2.0 | 1.5 |
| Diamine | 0 | 0.6 | 0.6 | 0.4 |
| Magnesium salt | 0.3 | 0.1 | 0 | 0 |
| Perfume | 0.5 | 1.0 | 1.5 | 1.5 |
| Finishing Agents | qs | qs | qs | qs |

LAS - Sodium Linear alkylphenyl sulfonate; AS - Sodium or potassium or monoethanolamine C₁₂ or C₁₂₋₁₃ or C₁₂₋₁₄ alkyl sulfate; AE(1)S - Sodium or potassium or monoethanolamine neutralized C₁₂ or C₁₂₋₁₃ or C₁₂₋₁₄ alkyl ethoxy(1) Sulfate; MES - C₁₂₋₁₄ methyl ester sulfonate; CAPB- Cocoamidopropyl Betaine; CMEA- Cocomonoethanolamide; C11E9 - C11 ethoxylate (9); APG - C12-14 alkyl polyglucoside; Coco Amine Oxide - C₁₂₋₁₄ alkyl dimethyl amine oxide; Diamine - 1,3 cyclohexanediamine, 1,3 propane diamine, any C3 to C7 alkyl diamine; Magnesium salt - magnesium chloride, magnesium hydroxide or magnesium sulfate; Finishing Agents: preservatives, solvents, salts, dyes, buffers, processing aids, excipients, etc.

### Example 5: Shampoo Formulae Having a Bio-Based Linear Alkylphenyl Sulfonate (LAS) as Primary/Co-Surfactant

The mixture of bio-based C₁₀-C₁₄ linear alkylphenyl sulfonates is added to shampoo formulations, as shown in the below table. The bio-based C₁₀-C₁₄ linear alkylphenyl sulfonate has an average chain length of 10.9.

| Formulation/ Component | Typical | A | B | C |
|---|---|---|---|---|
| Surfactants | | | | |
| SLS | 1.5 | 1.5 | 1.5 | 1.5 |
| SLE(1)S | 10 | | 10 | 12 |
| LAS from Example 1d | | 10 | 2 | 2 |
| CMEA | 0.5 | | | |
| CAPB | 2 | 2 | | 1 |
| Benefit Agents | | | | |
| Guar Cationic Polymer | 0.25 | 0.25 | 0.25 | 0.25 |
| LP Silicone | 1.0 | 1.0 | 1.0 | 1.0 |
| ZPT | | | | 1.0 |
| Aesthetics | | | | |
| EGDS | 1.5 | 1.5 | 1.5 | 1.5 |
| Perfume | 1.5 | 1.5 | 1.5 | 1.5 |
| Finishing Agents | qs | qs | qs | qs |

SLS - Sodium Lauryl Sulfate; SLE(1)S- Sodium Laureth(1) Sulfate; CAPB- Cocoamidopropyl Betaine; CMEA- Cocomonoethanolamide; EGDS- Etheylene Glycol Distearate; Guar-Hydroxypropyltrimoinum guar (cationic); LP-Silicone - Large Particle (>20 um) silicone; PQ-10 Polyquat-10; ZPT- Zinc pyridinethione; Finishing Agents: preservatives, salts, buffers, processing aids, excipients, etc.

## Claims

1. A method of making a mixture of C₁₀-C₁₄ linear alkylphenyl sulfonates having a controlled total carbon atom distribution comprising the steps of
(a) dehydrating a C₁₀-C₁₄ alcohol mixture derived either from plant matter or a bioengineered microorganism to form a mixture of C₁₀-C₁₄ alkenes; wherein each alkene has a biobased content of at least 50%;
(b) optionally isolating the C₁₀-C₁₄ alkenes;
(c) alkylating benzene with the mixture of C₁₀-C₁₄ alkenes to form a mixture comprising C₁₀-C₁₄ linear alkylbenzenes; and
(d) sulfonating the mixture of C₁₀-C₁₄ linear alkylbenzenes to form a mixture comprising C₁₀-C₁₄ linear alkylphenyl sulfonates.

2. The method of claim 1, wherein the benzene has a biobased content of at least 50%.

## Patentansprüche

1. Verfahren zur Herstellung einer Mischung aus linearen C₁₀-C₁₄-Alkylphenylsulfonaten mit einer kontrollierten Gesamtkohlenstoffatomverteilung, umfassend die folgenden Schritte:
(a) Dehydrieren einer C₁₀-C₁₄-Alkoholmischung, die entweder von Pflanzenmaterial oder einem durch Biotechnik erzeugten Mikroorganismus stammt, zur Bildung einer Mischung aus C₁₀-C₁₄-Alkenen, wobei jedes Alken einen biobasierten Gehalt von mindestens 50 % aufweist;
(b) optional Isolieren der C₁₀-C₁₄-Alkene;
(c) Alkylieren von Benzol mit der Mischung von C₁₀-C₁₄-Alkenen unter Bildung einer Mischung, die lineare C₁₀-C₁₄-Alkylbenzole umfasst; und
(d) Sulfonieren der Mischung von linearen C₁₀-C₁₄-Alkylbenzolen unter Bildung einer Mischung, die lineare C₁₀-C₁₄-Alkylphenylsulfonate umfasst.

2. Verfahren nach Anspruch 1, wobei das Benzol einen biobasierten Gehalt von mindestens 50 % aufweist.

## Revendications

1. Procédé de fabrication d'un mélange de sulfonates d'alkylphényle linéaires en C₁₀ à C₁₄ possédant une distribution régulée en atomes de carbone totaux comprenant les étapes consistant à
(a) déshydrater un mélange d'alcools en C₁₀ à C₁₄ dérivé ou d'une matière végétale ou d'un micro-organisme obtenu par bio-ingénierie pour former un mélange d'alcènes en C₁₀ à C₁₄; dans lequel chaque alcène a une teneur d'origine biologique d'au moins 50 % ;
(b) éventuellement isoler les alcènes en C₁₀ à C₁₄;
(c) alkyler du benzène avec le mélange d'alcènes en C₁₀ à C₁₄ pour former un mélange comprenant des alkylbenzènes linéaires en C₁₀ à C₁₄; et
(d) sulfoner le mélange d'alkylbenzènes linéaires en C₁₀ à C₁₄ pour former un mélange comprenant des sulfonates d'alkylphényle linéaires en C₁₀ à C₁₄.

2. Procédé selon la revendication 1, dans lequel le benzène a une teneur d'origine biologique d'au moins 50 %.
